# EUROPEAN PATENT APPLICATION

(11) **EP 4 474 479 A1**
(43) Date of publication of application: **11.12.2024**
(21) Application number: 23178553.6
(22) Date of filing: 09.06.2023
(51) Int. Cl.: C12N 15/113, A61P 13/12, C07K 16/18, A61P 39/06

(54) **DNA-BINDING PROTEIN-A MODULATOR FOR THE TREATMENT OF RENAL ISCHEMIA/REPERFUSION INJURY**

(71) Applicant: Otto-von-Guericke-Universität Magdeburg, 39106 Magdeburg (DE)
(72) Inventor: MERTENS, Peter R., 39104 Magdeburg (DE); REICHARDT, Charlotte, 39104 Magdeburg (DE)

(57) **Abstract**

The present invention relates to a DNA-binding protein-A (DbpA) modulating compound for use in the treatment of an acute kidney injury (AKI). In addition, the present invention relates to a combination of a DbpA modulating compound and a drug different from a DbpA modulating compound for use in the treatment of an acute kidney injury (AKI).

## Description

The present invention relates to a DNA-binding protein-A (DbpA) modulating compound for use in the treatment of an acute kidney injury (AKI). In addition, the present invention relates to a combination of a DbpA modulating compound and a drug different from a DbpA modulating compound for use in the treatment of an acute kidney injury (AKI).

### BACKGROUND OF THE INVENTION

Cold shock proteins are evolutionarily conserved, with all members sharing the eponymous cold shock domain (Lindquist and Mertens, 2018; Wolffe, 1994). Most attention has been attributed to oncogenic cold shock protein activities involving cell transformation, matrix invasion and unlimited proliferation (Hohlfeld et al., 2018). These have been scrutinized in detail in breast cancer (Bargou et al., 1997; Dahl et al., 2009), prostate cancer (Giménez-Bonafé et al., 2004) and multiple myeloma (Chatterjee et al., 2008). Recent findings link JAK/ERK signaling with cold shock Y-box binding protein-1 (YB-1) in myeloproliferative neoplasia, suggesting YB-1 as a therapeutic target in JAK2-mutated leukemia (Jayavelu et al., 2020). Whereas YB-1 is ubiquitously expressed, its homolog DNA-binding protein-A (DbpA) is described to be predominantly expressed during embryogenesis (Lima et al., 2010) and restricted later on, except in smooth muscle cells and testis (Lu et al., 2006). DbpA is encoded by the gene *Ybx3* and exists in two differential spliced isoforms (DbpA a, DbpA_b) (Lu et al., 2006; Scott et al., 2017; Wolffe et al., 1992). YB-1 and DbpA belong to the class of RNA-binding proteins that regulate complex inflammatory steps in kidney diseases, as summarized recently (Seufert and Benzing, 2022). For YB-1, activities during acute kidney injury (AKI) (Dong et al., 2015; Wang et al., 2023), diabetic kidney disease (Kato et al., 2010), tubulointerstitial fibrosis (Hanssen et al., 2013), glomerulosclerosis (Gibbert et al., 2018) and mesangioproliferative glomerulonephritis (van Roeyen et al., 2005) are reported. For DbpA, less is known on its involvement in diseases and development (Balda and Matter, 2000; Lima et al., 2010). Previous work identified DbpA as a regulator of mesangial cell proliferation in IgA nephritis (Zhu et al., 2016). Until now, its activities in acute injury models, such as ischemia/reperfusion (IRI) were, however, unclear.

The most common form of AKI relates to transient oxygen-deficiency with low blood pressure (pre-renal AKI) or impaired blood supply (e.g. vasoconstriction with non-steroidal anti-inflammatory drugs; ischemia during kidney transplantation) (Klomjit and Ungprasert, 2022). IRI is characterized by deficiency of oxygen and nutrients over a certain period of time causing mitochondrial dysfunction, release of cytochrome C and reactive oxygen species (ROS) (Malek and Nematbakhsh, 2015). In addition, reperfusion leads to rapid influx of oxygen-rich blood into the ischemic tissue and is characterized by epithelial and endothelial cell damage, consecutive attraction of inflammatory immune cells and cell death. A recovery process partially counteracts tissue damage by replacing tubular cells via stem-cell mediated regeneration (De Chiara et al., 2022; Peired and Melica, 2021). The cells that are most vulnerable under these conditions are the metabolically active TECs.

The diagnosis of AKI is mainly based on increasing serum creatinine levels. The molecular mechanisms underlying injury patterns are closely linked to the high oxygen consumption rate of tubule cells. Their metabolic activity is closely related to activated cell stress programs and tubular cell injury. Episodes of acute renal cell stress are frequent events, either with acute blood loss in traumatic accidents or after childbirth. Other causes are episodes of hypotension with bacterial infection and sepsis as well as heart failure or arterial hypertension in aging patients.

Therapeutic interventions include medications that force diuresis. In addition, hypotension based on impaired fluid intake and negative water balance can lead to acute renal damage. AKI is a global public health problem, affecting 13.3 million patients with 1.7 million deaths per year.

In view of the above, it is important to understand the critical steps of cellular damage, especially for the proximal tubule cells, which are particularly oxygen-dependent and sensitive. Given these circumstances, there is also an urgent need for a therapeutic agent that limits tubular damage, inflammatory response, and organ fibrosis.

In the study of the present inventors, a model of transient renal ischemia was used. It shows that the contribution of the cold shock protein DbpA to tubular damage is immense. The present inventors have further demonstrated that the deletion of the cold shock protein DbpA in the carpus has protective function. In cells lacking the cold shock protein DbpA, the mitochondrial function and the antioxidant activity are set at levels that confer resistance to acute cellular damage.

Accordingly, the present inventors have identified DbpA as a therapeutic target for AKI. As one possible intervention of the targeted blockade of DbpA, they suggest the intravenous administration of antibodies directed against the protein, thus, preventing extracellular functions of the protein. In addition, the present inventors suggest the administration of mirco RNAs (miRNAs), specifically of miRNA-191. The miR-191 suppresses the expression of DbpA. Hence, preemptive DbpA targeting in situations with expected IRI, such as kidney transplantation or cardiac surgery, is able to preserve kidney function.

### SUMMARY OF THE INVENTION

In a first aspect, the present invention relates to a DNA-binding protein-A (DbpA) modulating compound for use in the treatment of an acute kidney injury (AKI).

In a second aspect, the present invention relates to a combination of a DbpA modulating compound and a drug different from a DbpA modulating compound for use in the treatment of an acute kidney injury (AKI).

In a third aspect, the present invention relates to a pharmaceutical composition comprising a DNA-binding protein-A (DbpA) according to the first aspect or a combination according to the second aspect for use in the treatment of an acute kidney injury (AKI).

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of" according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "disease", as used herein, refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune disease. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infectious, isolated symptoms, deviant behaviors and atypical variations of structure and function, while in other contexts and for other purposes these may be considered distinguishable categories. Diseases usually affect individuals not only physically, but also emotionally, as contracting and living with many diseases can alter one's perspective on life, and one's personality. In the context of the present invention, the disease is a disease accompanied

The present invention is about the treatment of an acute kidney injury (AKI) in patients.

The term "acute kidney injury (AKI) (also known as acute renal failure (ARF)", as used herein, refers to a sudden episode of kidney failure or kidney damage that happens within a few hours or a few days. AKI causes a build-up of waste products in the patient's blood and makes it hard for the patient's kidneys to keep the right balance of fluid in the body.
More specifically, AKI is a nonspecific clinical syndrome defined by a rapid decrease in glomerular filtration rate (GFR) over ≤7 days. The consensus definition, established in 2004, marked a turning point in the clinical management and research associated with this disease. There are now three widely used diagnostic criteria for AKI-the RIFLE (Risk, Injury, Failure, Loss, End-Stage Kidney Disease) criteria, AKIN (AKI Network) criteria and KDIGO (Kidney Disease: Improving Global Outcomes) criteria -although clinical adjudication remains an integral part of establishing a diagnosis. The most recent definition of AKI requires an absolute increase in serum creatinine (SCr) concentration by 0.3 mg/dL (26.5 µmol/L) within 48 h, a relative increase in SCr by 50% above its baseline value within the prior 7 days and/or <0.5 mL/kg/h of urine production for ≥6 h. Some contention surrounds the diagnostic use of urine output and SCr since these biomarkers have significant shortcomings and could, therefore, misguide clinicians with respect to what is occurring in the kidney in real-time.
AKI can also affect other organs such as the brain, heart, and lungs. Acute kidney injury is common in patients who are in the hospital, in intensive care units, and especially in older adults.
Signs and symptoms of acute kidney injury differ depending on the cause and may include: (i) too little urine leaving the body, (ii) swelling in legs, ankles, and around the eyes, (iii) fatigue or tiredness, (iv) shortness of breath, (v) confusion, (vi) nausea, (vii) seizures or coma in severe cases, and (viii) c chest pain or pressure. In some cases, AKI causes no symptoms and is only found through other tests done by your healthcare provider.

In the context of the present invention, prerenal causes of AKI ("prerenal AKI") are those that decrease effective blood flow to the kidney and cause a decrease in the glomerular filtration rate (GFR). Both kidneys need to be affected as one kidney is still more than adequate for normal kidney function. Notable causes of prerenal AKI include low blood volume (e.g. dehydration), low blood pressure, heart problems (leading to cardiorenal syndrome), hepatorenal syndrome in the context of liver cirrhosis, and local changes to the blood vessels supplying the kidney. The latter include renal artery stenosis, or the narrowing of the renal artery which supplies the kidney with blood, and renal vein thrombosis, which is the formation of a blood clot in the renal vein that drains blood from the kidney.

The term "intrarenal/intrinsic AKI", as used herein, refers to disease processes which directly damage the kidney itself. Intrarenal/intrinsic AKI can be due to one or more of the kidney's structures including the glomeruli, kidney tubules, or the interstitium. Common causes of each are glomerulonephritis, acute tubular necrosis (ANT), and acute interstitial nephritis (AIN), respectively. Other causes of intrinsic AKI are rhabdomyolysis and tumor lysis syndrome. Certain medication classes such as calcineurin inhibitors can also directly damage the tubular cells of the kidney and result in a form of intrinsic AKI.

The term "postrenal AKI", as used herein, refers to AKI caused by disease states downstream of the kidney and most often occurs as a consequence of urinary tract obstruction. This may be related to benign prostatic hyperplasia, kidney stones, obstructed urinary catheter, bladder stones, or cancer of the bladder, ureters, or prostate.

The term "Ischaemia reperfusion injury (IRI)", as used herein, refers to a common mode of injury for multiple organs including the kidney, heart and brain. Renal IRI may lead to acute kidney injury (AKI) in patients and no specific treatment is available. AKI as a result of IRI has a complicated pathogenesis involving both the innate and adaptive immune response. Experimental models that accurately and reproducibly recapitulate renal IRI are crucial in dissecting the pathophysiology of AKI and the development of novel therapeutic agents. In the study of the present inventors, a model of transient renal ischemia was used. It shows that the contribution of the cold shock protein DbpA to tubular damage is immense. The present inventors have further demonstrated that the deletion of the cold shock protein DbpA in the carpus has protective function. In cells lacking the cold shock protein DbpA, the mitochondrial function and the antioxidant activity are set at levels that confer resistance to acute cellular damage. Accordingly, the present inventors have identified DbpA as a therapeutic target for AKI.

The term "treatment", in particular "therapeutic treatment", as used herein, refers to any therapy which improves the health status and/or prolongs (increases) the lifespan of an individual suffering from a disease or condition, in particular a tumor. Said therapy may eliminate the disease or condition in an individual, arrest or slow the development of a disease in an individual, inhibit or slow the development of a disease in an individual, decrease the frequency or severity of symptoms in an individual, and/or decrease the recurrence in an individual who currently has or who previously has had a disease. The present invention relates to the treatment of AKI with DNA-binding protein-A (DbpA) modulators.

The term "DNA-binding protein-A (DbpA)", as used herein, refers to a protein that belongs to the cold shock protein family. Cold shock proteins are evolutionarily conserved, with all members sharing an eponymous cold shock domain. Cold shock proteins have known functions in cell cycling, transcription, translation, and tight junction communication. DbpA is encoded by the *Ybx3* gene. DbpA has two splice isoforms, namely DbpA_a and DbpA_b. In chronic nephritis, DbpA is upregulated, however the activities of DbpA in acute injury models, such as ischemia/reperfusion (IRI), were, until now, unclear. The study of the present inventors, in which a model of transient renal ischemia was used, could show that the contribution of the cold shock protein DbpA to tubular damage is immense. The present inventors have further demonstrated that the deletion of the cold shock protein DbpA in the carpus has protective function. In cells lacking the cold shock protein DbpA, the mitochondrial function and the antioxidant activity are set at levels that confer resistance to acute cellular damage. Accordingly, the present inventors have identified DbpA as a therapeutic target for AKI. DbpA expression/function can be regulated using a DbpA modulating compound.

The term "DbpA modulating compound", as used herein, refers to a molecule which is able to influence the level of DbpA within a bodily cell/at cellular level. The presence of a DbpA modulating compound specifically decreases the level of DbpA within a cell, inhibits DbpA within a cell, and/or blocks DbpA function within a cell. Preferably, the DbpA modulating compound is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities. More preferably, the DbpA modulating compound is a miRNA or an antibody.
As mentioned above, the DNA-binding protein-A (DbpA) has two splice isoforms, namely DbpA_a and DbpA_b. Thus, the DbpA modulating compound may be directed to one of the splice isoforms, namely DbpA_a or DbpA_b, or to both of the splice isoforms, namely DbpA_a and DbpA_b. Even more preferably, the DbpA modulating compound is a miRNA or an antibody specific for the splice isoforms DbpA_a and/or DbpA_b.

The DbpA modulating compound may be a DbpA binding or inhibiting molecule.

The term "DbpA binding molecule", as used herein, refers to a compound which is able to bind to or hybridize with DbpA. The term "DbpA inhibiting molecule", as used herein, refers to a compound which is able to inhibit DbpA's function within a bodily cell/at cellular level. Said DbpA binding molecule can decrease the expression of DbpA or block DbpA. The DbpA activity can be inhibited or significantly reduced by using said DbpA binding molecule. Due to its binding, the biochemical and biological function of DbpA is inhibited or at least partially inhibited. Preferably, the DbpA binding/inhibiting molecule is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities. More preferably, the DbpA binding/inhibiting compound is a miRNA or an antibody. Even more preferably, the DbpA binding/inhibiting compound is a miRNA or an antibody specific for the splice isoforms DbpA_a and/or DbpA_b.

The term "miRNA" (the designation "microRNA" is also possible), as used herein, refers to a single-stranded RNA molecule. The miRNA may be a molecule of 10 to 50 nucleotides in length. The miRNAs regulate gene expression and are encoded by genes from whose DNA they are transcribed but miRNAs are not translated into protein (i.e. miRNAs are non-coding RNAs). The genes encoding miRNAs are longer than the processed mature miRNA molecules. The miRNA is initially transcribed as a longer precursor molecule (>1000 nucleotides long) called a primary miRNA transcript (pri-miRNA). Pri-miRNAs have hairpin structures that are processed by the Drosha enzyme (as part of the microprocessor complex). After Drosha processing, the pri-miRNAs are only 60-100 nucleotides long, and are called precursor miRNAs (pre-miRNAs). At this point, the pre-miRNA is exported to the cytoplasm, where it encounters the Dicer enzyme. Dicer cuts the miRNA in two, resulting in duplexed miRNA strands. Traditionally, only one of these miRNA arms was considered important in gene regulation: the arm that is destined to be loaded into the RNA-induced silencing complex (RISC), and occurs at a higher concentration in the cell. This is often called the "guide" strand and is designated as miR. The other arm is called the "minor miRNA" or "passenger miRNA", and is often designated as miR*. It was thought that passenger miRNAs were completely degraded, but deep sequencing studies have found that some minor miRNAs persist and in fact have a functional role in gene regulation. Due to these developments, the naming convention has shifted. Instead of the miR/miR* name scheme, a miR-5p/miR-3p nomenclature has been adopted. By the new system, the 5' arm of the miRNA is always designated miR-5p and the 3' arm is miR-3p. The present nomenclature is as follows: The prefix "miR" is followed by a dash and a number, the latter often indicating order of naming. For example, hsa-miR-16 was named and likely discovered prior to hsa-miR-342. A capitalized "miR-" refers to the mature forms of the miRNA (e.g. hsa-miR-16-5p and hsa-miR-16-3p), while the uncapitalized "mir-" refers to the pre-miRNA and the pri-miRNA (e.g. hsa-mir-16), and "MIR" refers to the gene that encodes them. However, as this is a recent change, literature will often refer to the original miR/miR* names. After processing, the duplexed miRNA strands are loaded onto an Argonaute (AGO) protein to form a precursor to the RISC. The complex causes the duplex to unwind, and the passenger RNA strand is discarded, leaving behind a mature RISC carrying the mature, single stranded miRNA. The miRNA remains part of the RISC as it silences the expression of its target genes. While this is the canonical pathway for miRNA biogenesis, a variety of others have been discovered. These include Drosha-independent pathways (such as the mirtron pathway, snoRNA-derived pathway, and shRNA-derived pathway) and Dicer-independent pathways (such as one that relies on AGO for cleavage, and another which is dependent on tRNaseZ). The miRNA is preferably used to bind to/neutralize DbpA.

The terms "antibody", "immunoglobulin", "Ig" and "Ig molecule" are used interchangeably herein. They refer to Y-shaped proteins that are produced by the immune system to help stop intruders from harming the body. When an intruder enters the body, the immune system springs into action. These invaders, which are called antigens, can be viruses, bacteria, or other chemicals. When an antigen is found in the body, the immune system will create antibodies to mark the antigen for the body to destroy. The terms are used in their broadest sense and include monoclonal antibodies (including full length monoclonal antibodies), polyclonal antibodies, single chain antibodies, and multispecific antibodies (e.g. bispecific antibodies). The term "antibody fragment", as used herein, refers to a polypeptide that includes at least one immunoglobulin variable domain or immunoglobulin variable domain sequence and which specifically binds a specific antigen. An antibody fragment can comprise an antibody or a polypeptide comprising an antigen-binding domain of an antibody. An antibody fragment can comprise a monoclonal antibody or a polypeptide comprising an antigen-binding domain of a monoclonal antibody. For example, an antibody can include a heavy (H) chain variable region and a light (L) chain variable region. An antibody may include two heavy (H) chain variable regions and two light (L) chain variable regions.

The term "antibody fragment" encompasses antigen-binding fragments of antibodies such as single chain antibodies, Fab fragments, F(ab')₂, Fv fragments and scFv. An antibody can have the structural features of IgA, IgG, IgE, IgD, IgM (as well as subtypes and combinations thereof). Antibodies can be from any source, including mouse, rabbit, pig, rat, and primate (human and non-human primate), whereby the antibodies have to be humanized before administered to a patient. Methods to humanize antibodies are well known in the art. The antibody or a fragment thereof is preferably used to bind to/neutralize DbpA.

DbpA blocking/inhibition can be achieved using RNA interference (RNAi) technology. RNA interference uses typically small interfering RNAs (siRNAs) and/or short hairpin RNAs (shRNAs). The administration of such molecules (e.g. siRNAs, shRNAs) leads to a decrease of the level of DbpA within the cell.

The term "siRNA", as used herein, refers to typically double-stranded RNA molecules (dsRNA) that mediate the targeted cleavage of a RNA transcript via a RNA-induced silencing complex (RISC) pathway. siRNA molecules interfere with the expression of specific genes with complementary nucleotide sequences by degrading mRNA after transcription.

The terms "short hairpin RNA (shRNA)" or "small hairpin RNA (shRNA)", as used herein, refer to an artificial RNA molecule with a tight hairpin turn that can be used to silence target gene expression via RNA interference (RNAi). In particular, the shRNAs comprise a hairpin structure (also called stem loop). In a preferred embodiment, shRNAs comprise a short antisense strand, followed by a nucleotide loop of about 5 to about 9 nucleotides, and the analogous sense strand. Alternatively, the sense strand may precede the nucleotide loop structure and the antisense strand may follow.

The administration of the above-mentioned DbpA modulating molecules, specifically DbpA binding molecules, (e.g. miRNA, siRNA, shRNA) leads to a decrease in expression of DbpA, inhibition of Dbp, and/or blocking of DbpA. In some embodiments, DbpA expression is decreased, DbpA expression is inhibited, and/or DbpA blocking is achieved for an extended duration, e.g. at least one week, two weeks, three weeks, or four weeks or longer. For example, in certain instances, DbpA expression, DbpA inhibition, and/or DbpA function is suppressed by at least 5%, preferably at least 10%, more preferably at least 15%, more preferably at least 20%, more preferably at least 25%, more preferably at least 30%, more preferably at least 40%, more preferably at least 50%, more preferably at least 60%, more preferably at least 70%, more preferably at least 80%, more preferably at least 90%, even more preferably at least 95%, 98%, or even 100% by administration of the DbpA modulating molecules, specifically DbpA binding molecules, described herein. The degree of identity of the antisense sequences to the targeted sequence should be at least 85%, preferably at least 90%, more preferably at least 95%, more preferably at least 98%, in particular 100%.

The DbpA modulating compound may be administered topically, intravenously, intradermally, intraarterially, intraperitoneally, intralesionally, intracranially, intraarticularly, intraprostaticaly, intrapleurally, intratracheally, intraocularly, intranasally, intravitreally, intravaginally, intrarectally, intramuscularly, subcutaneously, subconjunctivally, intravesicularly, mucosally, intrapericardially, intraumbilically, orally, by inhalation, by injection, by infusion, by continuous infusion, by localized perfusion bathing target cells directly, via a catheter, or via a lavage.

The DbpA modulating compound may also be administered by providing a delivery system selected from the group consisting of an expression construct, e.g. a (viral) vector, a liposome, a polymer-mediated delivery system, a conjugate delivery system, an exosome, a microsponge, and a nanoparticle, e.g. a gold nanoparticle.

The DbpA modulating compound may be administered in the form of any suitable pharmaceutical composition. Said pharmaceutical composition may further comprise pharmaceutical acceptable carriers, diluents, and/or excipients.

The term "systemic administration", a used herein, refers to the administration of the DbpA modulating compound such that said compound becomes widely distributed in the body of a patient in significant amounts and develops a biological effect. Typical systemic routes of administration include administration by introducing the DbpA modulating compound directly into the vascular system or oral, pulmonary, or intramuscular administration wherein the DbpA modulating compound enters the vascular system and is carried to one or more desired site(s) of action via the blood. The systemic administration may be by parenteral administration.

The term "parenteral administration", as used herein, refers to the administration of the DbpA modulating compound such that said compound does not pass the intestine. Parenteral administration includes intravenous administration, subcutaneous administration, intradermal administration, or intraarterial administration, but is not limited thereto.

The pharmaceutical composition according to the present invention is generally applied in a pharmaceutically effective amount. The term "pharmaceutically effective amount", as used herein, refers to the amount which achieves a desired reaction or a desired effect alone or together with further doses. In case of the treatment of a particular disease, the desired reaction preferably relates to an inhibition of the course of the disease. This comprises slowing down the progress of the disease and, in particular, interrupting or reversing the progress of the disease. The desired reaction in a treatment of a disease may also be a delay of the onset or a prevention of the onset of the disease. An effective amount of the compounds or compositions described herein will depend on the condition to be treated, the severeness of the disease, the individual parameters of the patient, including age, physiological condition, size, and weight, the duration of treatment, the type of an accompanying therapy (if present), the specific route of administration, and similar factors. Accordingly, the doses of the compounds or compositions described herein may depend on various of such parameters. In case that a reaction in the patient/subject is insufficient with an initial dose, higher doses (or effectively higher doses achieved by a different, more localized route of administration) may be used.

As mentioned above, the pharmaceutical composition of the present invention may further comprise pharmaceutical acceptable carriers, diluents, and/or excipients.

The term "excipient", as used herein, is intended to indicate all substances in a pharmaceutical composition which are not active ingredients such as binders, lubricants, thickeners, surface active agents, preservatives, emulsifiers, buffers, flavoring agents, or colorants.

The term "diluent", as used herein, relates to a diluting and/or thinning agent. Moreover, the term "diluent" includes a solution, suspension (e.g. liquid or solid suspension) and/or media.

The term "carrier", as used herein, relates to one or more compatible solid or liquid fillers, which are suitable for an administration, e.g. to a human. The term "carrier" relates to a natural or synthetic organic or inorganic component which is combined with an active component in order to facilitate the application of the active component. Preferably, carrier components are sterile liquids such as water or oils, including those which are derived from mineral oil, animals, or plants, such as peanut oil, soy bean oil, sesame oil, sunflower oil, etc. Salt solutions and aqueous dextrose and glycerin solutions may also be used as aqueous carrier compounds.

Pharmaceutically acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include, for example, magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Examples of suitable diluents include ethanol, glycerol, and water.

Pharmaceutical carriers, diluents, and/or excipients can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose, and polyethylene glycol. Examples of suitable lubricants include sodium oleate, sodium stearate, magnesium stearate, sodium benzoate, sodium acetate, sodium chloride, and the like. Preservatives, stabilizers, dyes, and even flavoring agents may be provided in the pharmaceutical composition. Examples of preservatives include sodium benzoate, sorbic acid, and esters of p-hydroxybenzoic acid. Antioxidants and suspending agents may be also used.

The term "patient", as used herein, refers to any subject suffering from an acute kidney injury (AKI). The patient may be treated and the response to said treatment may be evaluated. The patient may be any mammal, including both a human and another mammal, e.g. an animal such as a rabbit, mouse, rat, or monkey. Human subjects as patients are particularly preferred.

### Embodiments of the invention

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

In the study of the present inventors, a model of transient renal ischemia was used. It shows that the contribution of the cold shock protein DbpA to tubular damage is immense. The present inventors have further demonstrated that the deletion of the cold shock protein DbpA in the carpus has protective function. In cells lacking the cold shock protein DbpA, the mitochondrial function and the antioxidant activity are set at levels that confer resistance to acute cellular damage.

Accordingly, the present inventors have identified DbpA as a therapeutic target for AKI. As one possible intervention of the targeted blockade of DbpA, they suggest the intravenous administration of antibodies directed against the protein, thus, preventing extracellular functions of the protein. In addition, the present inventors suggest the administration of mirco RNAs (miRNAs), specifically of miRNA-191. The miR-191 suppresses the expression of DbpA. Hence, preemptive DbpA targeting in situations with expected IRI, such as kidney transplantation or cardiac surgery, is able to preserve kidney function.

Thus, in a first aspect, the present invention relates to a DNA-binding protein-A (DbpA) modulating compound for use in the treatment of an acute kidney injury (AKI). Specifically, the DbpA modulating compound is used to treat AKI in a patient. The patient is preferably a mammal, more preferably a human.

Particularly, the DbpA modulating compound is able to influence the level of DbpA within a cell of a patient. The presence of a DbpA modulating compound specifically decreases the level of DbpA within a cell, inhibits DbpA within a cell, and/or blocks DbpA function within a cell.

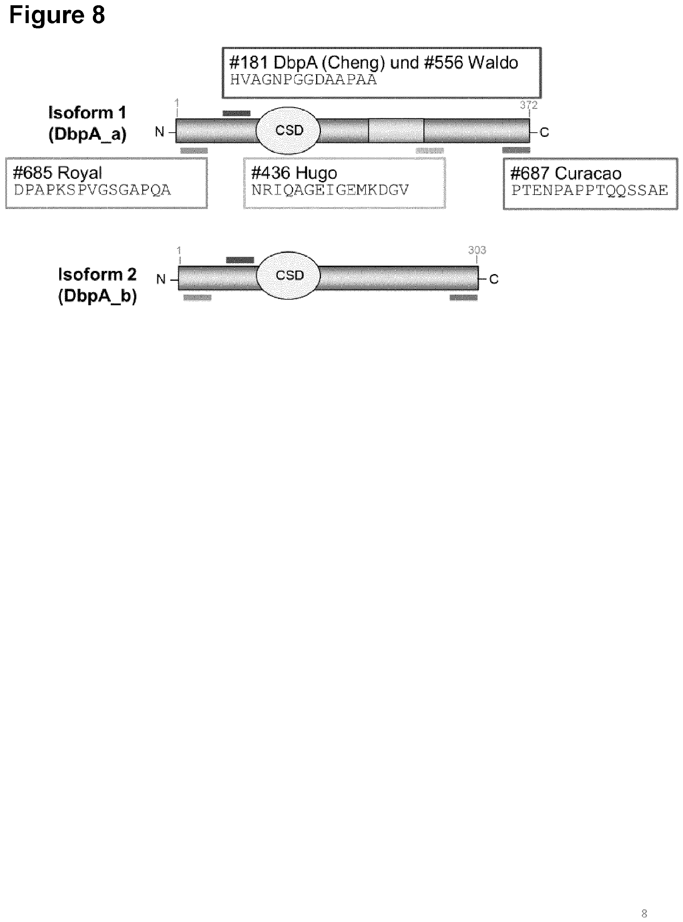

In one preferred embodiment, the DbpA modulating compound is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities. Preferably, the DbpA modulating compound is a (human) miRNA, or an (a human) antibody or a fragment thereof. More preferably, the DbpA modulating compound is miRNA-191 (e.g. human hsa-miRNA-191-5p or Mus musculus mmu-miR-191-5p, both have the sequence: CAACGGAAUCCCAAAAGCAGCUG (SEQ ID NO: 1)) or the antibody is an anti-DbpA antibody. Preferred anti-DbpA antibodies specifically bind to the epitope sequences shown in **Figure 8****.** The miRNA-191 binds/targets the 3'UTR of the mRNA of DbpA. Other miRNAs similarly interfering with DbpA synthesis may also be used.
As mentioned above, the DNA-binding protein-A (DbpA) has two splice isoforms, namely DbpA_a and DbpA_b. Thus, the DbpA modulating compound may be directed to one of the splice isoforms, namely DbpA_a or DbpA_b, or to both of the splice isoforms, namely DbpA_a and DbpA_b. Specifically, the DbpA modulating compound is a miRNA, or an antibody or a fragment thereof specific for the splice isoforms DbpA_a and/or DbpA_b.

In one more preferred embodiment, the DbpA modulating compound is a DbpA binding/inhibiting molecule. The DbpA binding/inhibiting molecule is able to bind to, to interact with, or to hybridize with DbpA. The DbpA binding molecule can decrease the expression of DbpA or block DbpA. The DbpA activity can be inhibited or significantly reduced by using said DbpA binding molecule. Due to its binding, the biochemical and biological function of DbpA is inhibited or at least partially inhibited. Preferably, the DbpA binding/inhibiting molecule is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities. More preferably, the DbpA binding/inhibiting compound is a (human) miRNA, or an (a human) antibody or a fragment thereof. Even more preferably, the DbpA binding/inhibiting compound is miRNA-191 (e.g. human hsa-miRNA-191-5p or Mus musculus mmu-miR-191-5p, both have the sequence: CAACGGAAUCCCAAAAGCAGCUG (SEQ ID NO: 1)) or the antibody is an anti-DbpA antibody. Preferred anti-DbpA antibodies specifically bind to the epitope sequences shown in **Figure 8****.** The miRNA-191 binds/targets the 3'UTR of the mRNA of DbpA. Other miRNAs similarly interfering with DbpA synthesis may also be used.
As mentioned above, the DNA-binding protein-A (DbpA) has two splice isoforms, namely DbpA_a and DbpA_b. Thus, the DbpA binding/inhibiting compound may be directed to one of the splice isoforms, namely DbpA_a or DbpA_b, or to both of the splice isoforms, namely DbpA_a and DbpA_b. Specifically, the DbpA binding/inhibiting compound is a miRNA, or an antibody or a fragment thereof specific for the splice isoforms DbpA_a and/or DbpA_b.

The administration of the above-mentioned DbpA modulating compound, specifically DbpA binding/inhibiting molecule, (e.g. miRNA, siRNA, shRNA) leads to a decrease in expression of DbpA, inhibition of Dbp, and/or blocking of DbpA. In some embodiments, DbpA expression is decreased, DbpA expression is inhibited, and/or DbpA blocking is achieved for an extended duration, e.g. at least one week, two weeks, three weeks, or four weeks or longer. For example, in certain instances, DbpA expression, DbpA inhibition, and/or DbpA function is suppressed by at least 5%, preferably at least 10%, more preferably at least 15%, more preferably at least 20%, more preferably at least 25%, more preferably at least 30%, more preferably at least 40%, more preferably at least 50%, more preferably at least 60%, more preferably at least 70%, more preferably at least 80%, more preferably at least 90%, even more preferably at least 95%, 98%, or even 100% by administration of the DbpA modulating molecules, specifically DbpA binding molecules, described herein. The degree of identity of the antisense sequences to the targeted sequence should be at least 85%, preferably at least 90%, more preferably at least 95%, more preferably at least 98%, in particular 100%.

The DbpA modulating compound, specifically binding/inhibiting molecule, may be administered topically, intravenously, intradermally, intraarterially, intraperitoneally, intralesionally, intracranially, intraarticularly, intraprostaticaly, intrapleurally, intratracheally, intraocularly, intranasally, intravitreally, intravaginally, intrarectally, intramuscularly, subcutaneously, subconjunctivally, intravesicularly, mucosally, intrapericardially, intraumbilically, orally, by inhalation, by injection, by infusion, by continuous infusion, by localized perfusion bathing target cells directly, via a catheter, or via a lavage.

The DbpA modulating compound, specifically binding/inhibiting molecule may also be administered by providing a delivery system selected from the group consisting of an expression construct, e.g. a (viral) vector, a liposome, a polymer-mediated delivery system, a conjugate delivery system, an exosome, a microsponge, and a nanoparticle, e.g. a gold nanoparticle.

In one even more preferred embodiment, the AKI is selected from the group consisting of prerenal or intrarenal AKI and postrenal AKI.

The first aspect of the present invention can alternatively be worded as follows: A method for treating an acute kidney injury (AKI) in an individual comprising the step of: administering (an effective amount of) a DNA-binding protein-A (DbpA) modulating compound to an individual in need thereof. The first aspect of the present invention can further alternatively be worded as follows: Use of a DNA-binding protein-A (DbpA) modulating compound for the manufacture of a medicament/drug for the treatment of an acute kidney injury (AKI).

In a second aspect, the present invention relates to a combination of a DbpA modulating compound and a drug different from a DbpA modulating compound for use in the treatment of an acute kidney injury (AKI).

The drug different from a DbpA modulating compound may be any compound which is (usually) used for treating an acute kidney injury (AKI). Preferably, the drug different from a DbpA modulating compound is selected from the group consisting of oxygen-free radical scavengers, angiotensin II and adenosine receptor antagonists, Alkaline phosphatase, sphingosine 1 phosphate analogues, and dipeptidylpeptidase-4 inhibitors. More preferably, the oxygen-free radical scavengers are selected from the group consisting of α-lipoic acid, curcumin, sodium-2-mercaptoethane sulphonate, propofol, and selenium.

Particularly, the DbpA modulating compound is able to influence the level of DbpA within a cell of a patient. The presence of a DbpA modulating compound specifically decreases the level of DbpA within a cell, inhibits DbpA within a cell, and/or blocks DbpA function within a cell.

In one preferred embodiment, the DbpA modulating compound is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities. Preferably, the DbpA modulating compound is a (human) miRNA, or an (a human) antibody or a fragment thereof. More preferably, the DbpA modulating compound is miRNA-191 (e.g. human hsa-miRNA-191-5p or Mus musculus mmu-miR-191-5p, both have the sequence: CAACGGAAUCCCAAAAGCAGCUG (SEQ ID NO: 1)) or the antibody is an anti-DbpA antibody. Preferred anti-DbpA antibodies specifically bind to the epitope sequences shown in **Figure 8****.** The miRNA-191 binds/targets the 3'UTR of the mRNA of DbpA. Other miRNAs similarly interfering with DbpA synthesis may also be used.

As mentioned above, the DNA-binding protein-A (DbpA) has two splice isoforms, namely DbpA_a and DbpA_b. Thus, the DbpA modulating compound may be directed to one of the splice isoforms, namely DbpA_a or DbpA_b, or to both of the splice isoforms, namely DbpA_a and DbpA_b. Specifically, the DbpA modulating compound is a miRNA, or an antibody or a fragment thereof specific for the splice isoforms DbpA_a and/or DbpA_b.

In one more preferred embodiment, the DbpA modulating compound is a DbpA binding/inhibiting molecule. Specifically, the DbpA binding/inhibiting molecule is able to bind to, to interact with, or to hybridize with DbpA. The DbpA binding molecule can decrease the expression of DbpA or block DbpA. The DbpA activity can be inhibited or significantly reduced by using said DbpA binding molecule. Due to its binding, the biochemical and biological function of DbpA is inhibited or at least partially inhibited. Preferably, the DbpA binding/inhibiting molecule is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities. More preferably, the DbpA binding/inhibiting compound is a (human) miRNA, or an (a human) antibody or a fragment thereof. Even more preferably, the DbpA binding/inhibiting compound is miRNA-191 (e.g. human hsa-miRNA-191-5p or Mus musculus mmu-miR-191-5p, both have the sequence: CAACGGAAUCCCAAAAGCAGCUG (SEQ ID NO:1)) or the antibody is an anti-DbpA antibody. Preferred anti-DbpA antibodies specifically bind to the epitope sequences shown in **Figure 8****.** The miRNA-191 binds/targets the 3'UTR of the mRNA of DbpA. Other miRNAs similarly interfering with DbpA synthesis may also be used.
As mentioned above, the DNA-binding protein-A (DbpA) has two splice isoforms, namely DbpA_a and DbpA_b.Thus, the DbpA binding/inhibiting compound may be directed to one of the splice isoforms, namely DbpA_a or DbpA_b, or to both of the splice isoforms, namely DbpA_a and DbpA_b. Specifically, the DbpA binding/inhibiting compound is a miRNA, or an antibody or a fragment thereof specific for the splice isoforms DbpA_a and/or DbpA_b.

The administration of the above-mentioned DbpA modulating compound, specifically DbpA binding/inhibiting molecule, (e.g. miRNA, siRNA, shRNA) leads to a decrease in expression of DbpA, inhibition of Dbp, and/or blocking of DbpA. In some embodiments, DbpA expression is decreased, DbpA expression is inhibited, and/or DbpA blocking is achieved for an extended duration, e.g. at least one week, two weeks, three weeks, or four weeks or longer. For example, in certain instances, DbpA expression, DbpA inhibition, and/or DbpA function is suppressed by at least 5%, preferably at least 10%, more preferably at least 15%, more preferably at least 20%, more preferably at least 25%, more preferably at least 30%, more preferably at least 40%, more preferably at least 50%, more preferably at least 60%, more preferably at least 70%, more preferably at least 80%, more preferably at least 90%, even more preferably at least 95%, 98%, or even 100% by administration of the DbpA modulating molecules, specifically DbpA binding molecules, described herein. The degree of identity of the antisense sequences to the targeted sequence should be at least 85%, preferably at least 90%, more preferably at least 95%, more preferably at least 98%, in particular 100%.

The DbpA modulating compound and the drug different from a DbpA modulating compound can be administered to a patient either together (in a composition or in separate compositions but given to the patient simultaneously or nearly simultaneous, for instance, within the hour of administration of the other compound/drug) or separately (e.g., administered in separate compositions and at different times, typically more than 2, 3, 4, 5, 6, 7, 8, 9, 10, 12, or more hours apart, or even via different routes). For example, the DbpA modulating compound may be administered up to 1 to 12 hours after the drug different from a DbpA modulating compound is administered. Alternatively, the drug different from a DbpA modulating compound may be administered up to 1 to 12 hours after the DbpA modulating compound is administered. Thus, the DbpA modulating compound and the drug different from a DbpA modulating compound, which are applied in combination to treat AKI, may be part of a composition (the same composition) or may be part of different compositions which is/are used for the treatment.

In one even more preferred embodiment, the AKI is selected from the group consisting of prerenal or intrarenal AKI and postrenal AKI.

As to further specific and preferred embodiments, it is referred to the first aspect of the present invention.

The second aspect of the present invention can alternatively be worded as follows: A method for treating an acute kidney injury (AKI) in an individual comprising the step of: administering (an effective amount of) a combination of a DbpA modulating compound and a drug different from a DbpA modulating compound to an individual in need thereof. The second aspect of the present invention can further alternatively be worded as follows: Use of a combination of a DbpA modulating compound and a drug different from a DbpA modulating compound for the manufacture of a medicament/drug for the treatment of an acute kidney injury (AKI).

In a third aspect, the present invention relates to a pharmaceutical composition comprising a DNA-binding protein-A (DbpA) according to the first aspect or a combination according to the second aspect for use in the treatment of an acute kidney injury (AKI).

In one preferred embodiment, the pharmaceutical composition further comprises pharmaceutical acceptable carriers, diluents, and/or excipients.

The pharmaceutical composition may be formulated for local administration or systemic administration. In particular, the local administration is by parenteral administration, e.g. by intravenous administration, subcutaneous administration, intradermal administration, intramuscularly administration, and the systemic administration is by intraarterial administration. In particular the composition is administered subcutaneously, intradermally, or intramuscularly. The composition may further comprise one or more pharmaceutically acceptable carriers, diluents, and/or excipients.

In one more preferred embodiment, the AKI is selected from the group consisting of prerenal or intrarenal AKI and postrenal AKI.

As to further specific and preferred embodiments, it is referred to the first or second aspect of the present invention.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following Figures are merely illustrative of the present invention and should not be construed to limit the scope of the invention as indicated by the appended claims in any way.

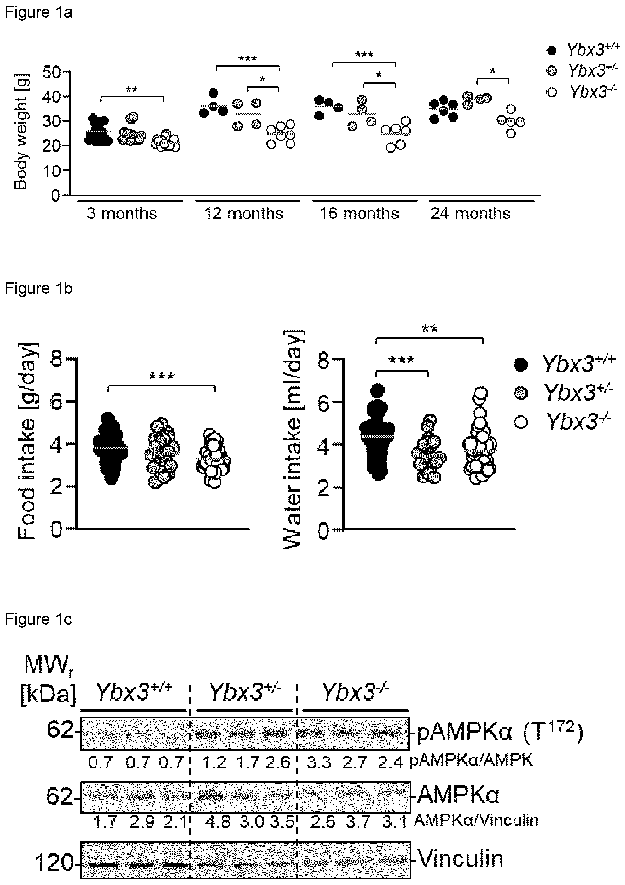

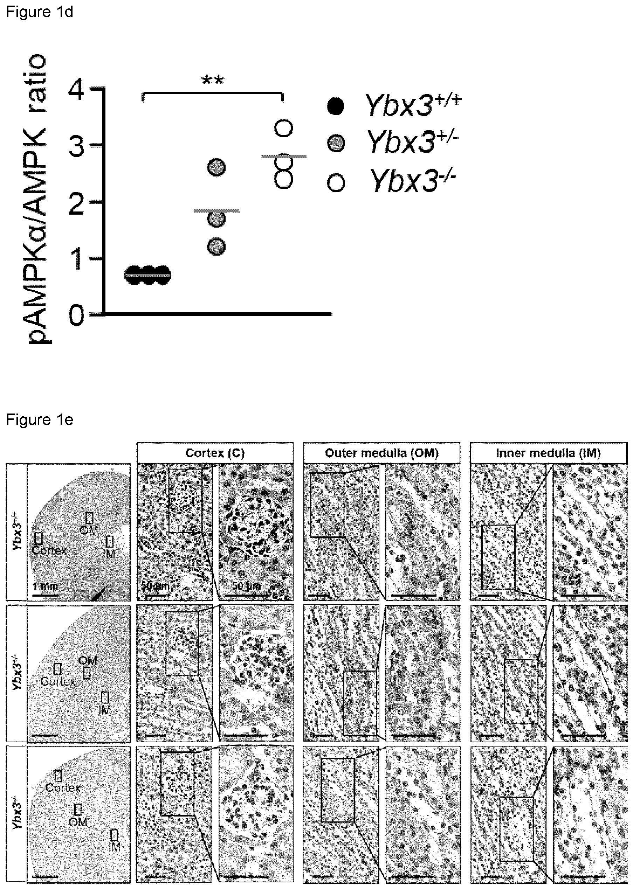

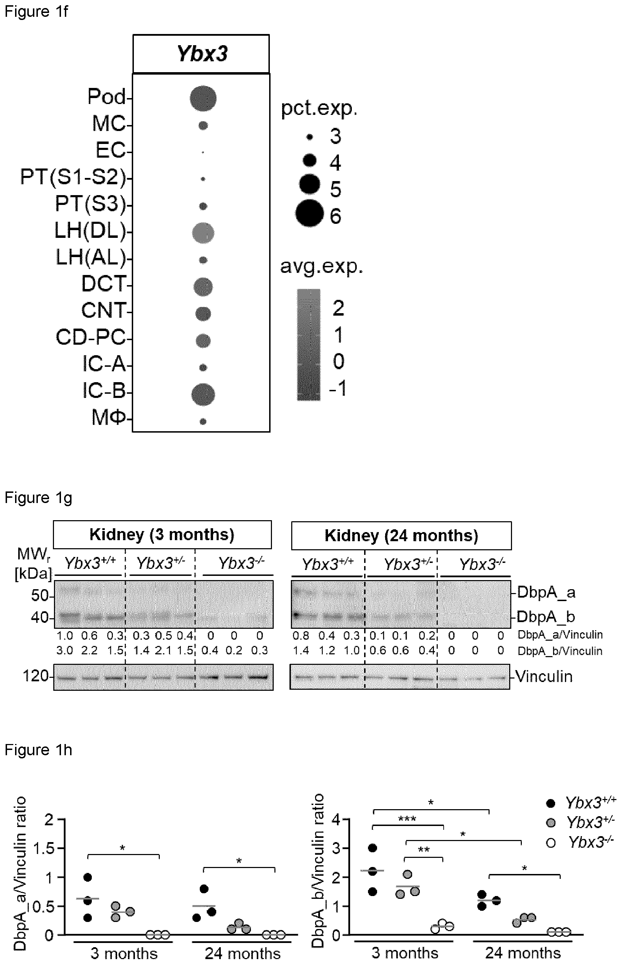

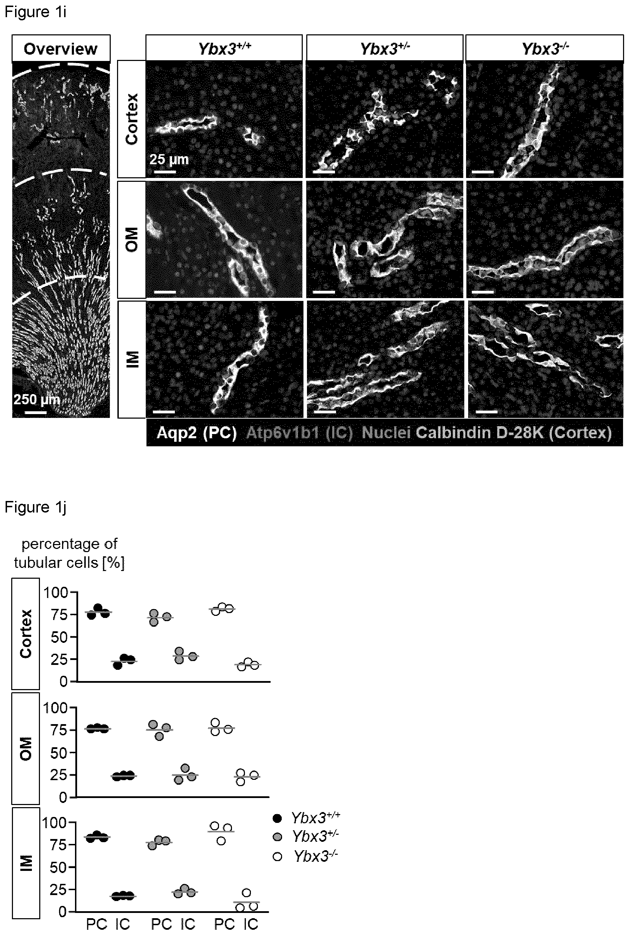

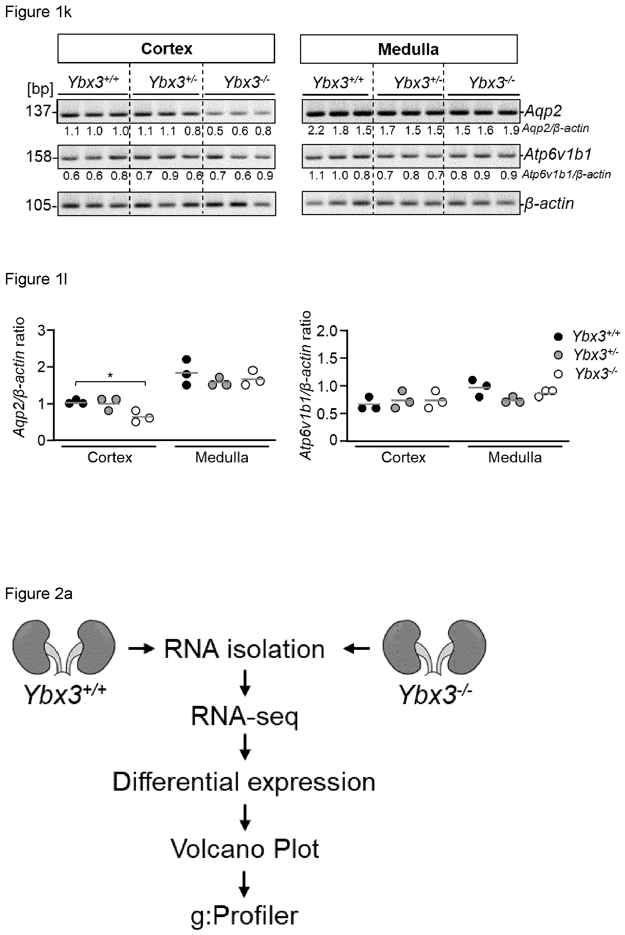

**Figure 1****: Phenotyping and long-term survival of mice with genetic Ybx3 deletion. (A)** In mice aged 3, 12, 16 and 24 months (n=4-12) body weights were significantly lower in Ybx3^{-/-} animals. Equal numbers of males and females were analyzed. **(B)** Food and water intake was reduced in Ybx3^{-/-} animals (age: 3 month, n=22-61). **(C)** Given the lowered body weight and reduced food/water intake, Western blot analyses for AMP-activated protein kinase α (AMPKα) as a key enzyme in energy metabolism were performed with kidney lysates from mice aged 10-12 weeks. **(D)** The pAMPKα (T¹⁷²)/AMPKα ratio was significantly higher in Ybx3^{+/-} and Ybx3^{-/-} compared to wild type animals. **(E)** Immunohistochemistry of kidney tissue from 3 months old wild type mice with the anti-C-terminal antibody demonstrated DbpA immunopositive staining in smooth muscle cells from small vessels, individual distal tubular cells, glomerular podocytes and mesangial cells. Ybx3^{+/-} and Ybx3^{-/-} animals exhibited lower or absent DbpA expression in the respective cells. Scale bars, 1 mm or 50 µm. **(F)** Immunohistochemistry findings matched with single cell sequencing data sets (Healthy Mouse Dataset; (Wu et al., 2019)) that revealed abundant Ybx3 transcripts in podocytes and distal tubular cells. Diameters of circles represent the number of positive cells and the color changes according to Ybx3 transcript numbers. **(G)** By means of a polyclonal anti-C-terminal antibody, two DbpA isoforms (DbpA a, DbpA_b) were detected in kidney tissue lysates. **(H)** The DbpA expression levels were lower at 24 months compared to early time points. DbpA_b expression was predominant in kidney lysates of young and old Ybx3^{+/+} and Ybx3^{+/-} animals, whereas in Ybx3^{-/-} animals both isoforms were not detectable. **(I)** Immunofluorescence staining for aquaporin 2 (Aqp2) as marker protein for principal cells (PC) and ATPase H+ transporting V1 subunit B1 (Atp6v1b1) as marker protein for intercalating cells (IC) with (**J**) quantification of relative cell numbers [%] within the cortex, OM and IM revealed no differences in the examined wild type, Ybx3^{+-/-} and Ybx3^{-/-} animals. Scale bars, 250 µm or 25 µm. (**K** and **L**) Semiquantitative PCR with cortex but not medulla tissue yielded lowered copy numbers for Aqp2 transcripts in Ybx3^{-/-} animals. Atp6v1b1 transcript numbers were similar in all strains. Abbreviations: podocyte (Pod), mesangial cell (MC), endothelial cell (EC), proximal tubule (PT), loop of Henle descending loop (LH(DL)), loop of Henle ascending loop (LH(AL)), distal convoluted tubule (DCT), connecting tubule (CNT), collecting duct-principal cell (CD-PC), intercalated cell type A (IC-A), intercalated cell type B (IC-B), macrophage (MΦ). Data were represented as mean values. All results were confirmed in triplicates by at least two independent experiments. One-way ANOVA followed by Tukey post-test was used for three group comparisons with ^{∗}p<0.05, ^{∗∗}p<0.01, ^{∗∗∗}p<0.001.

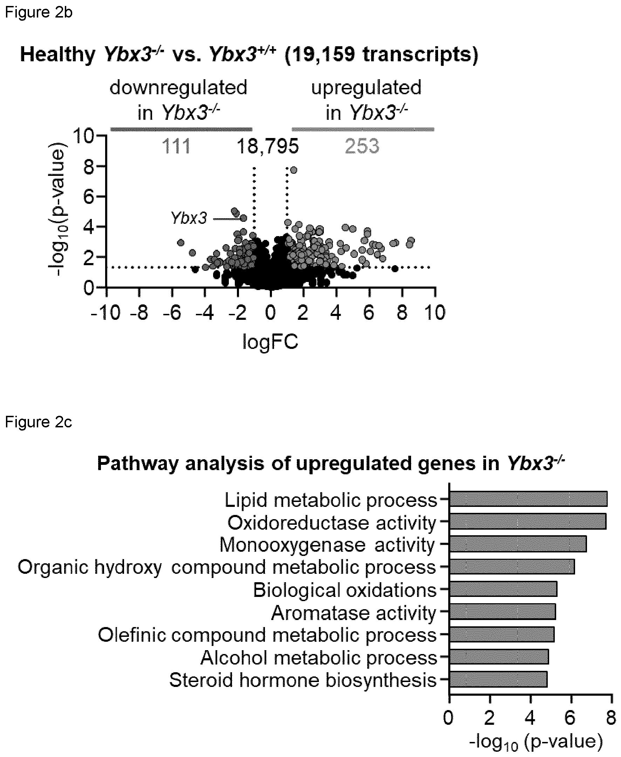

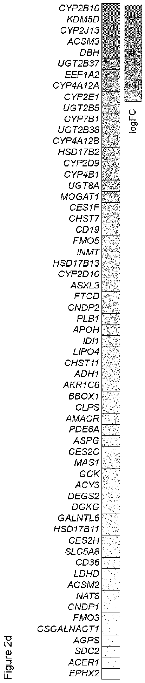

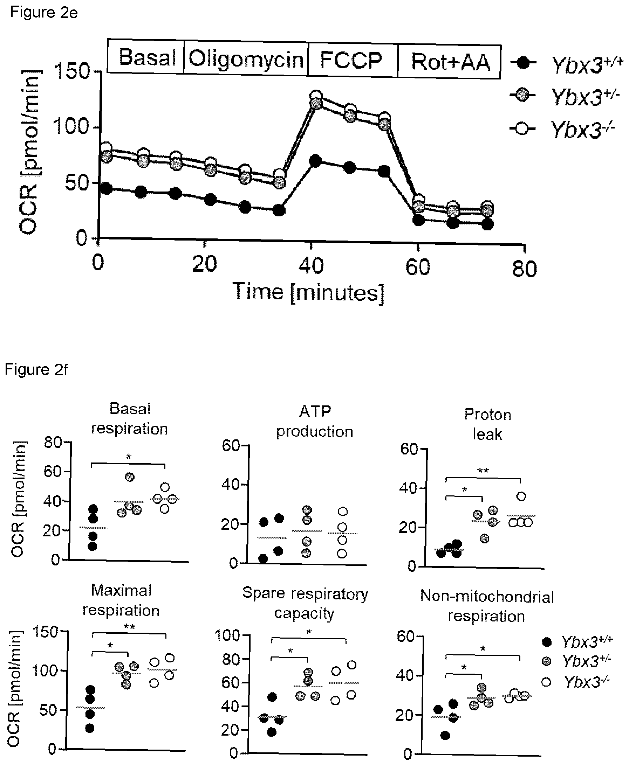

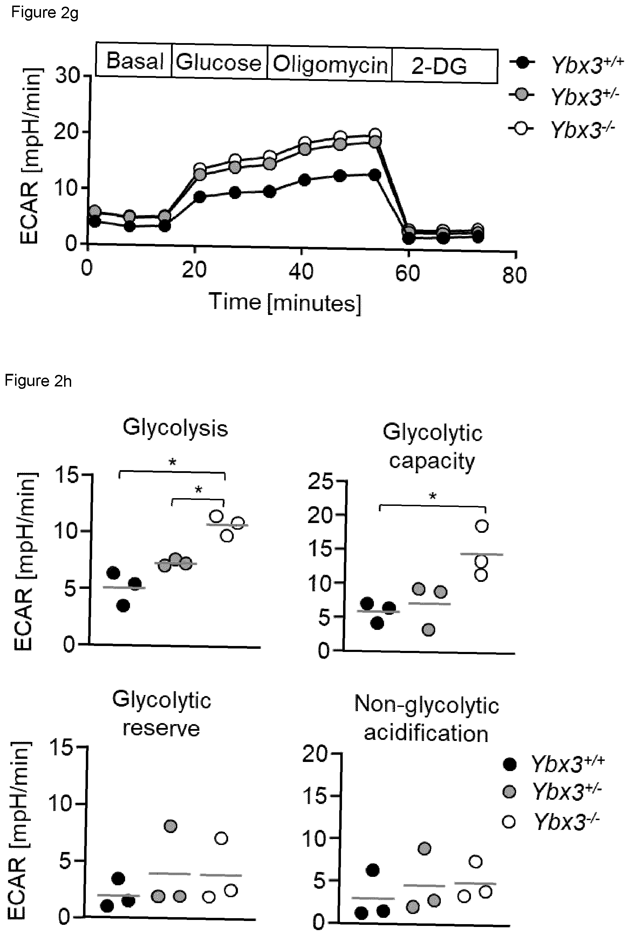

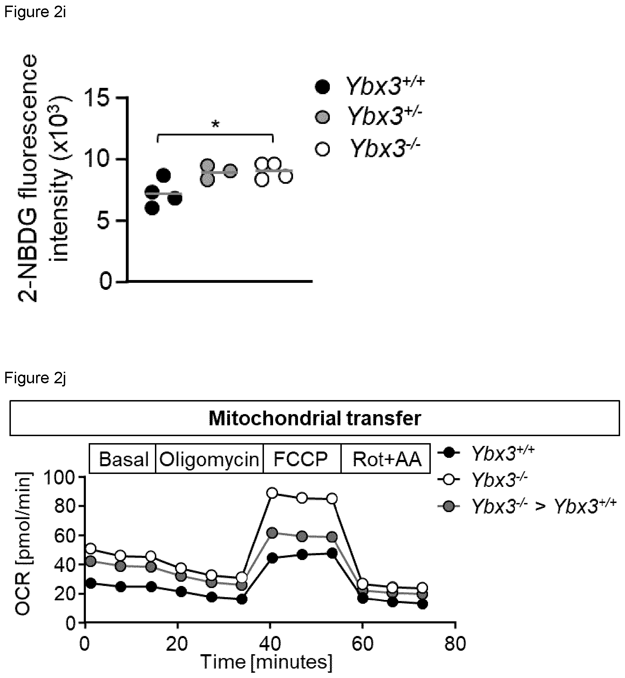

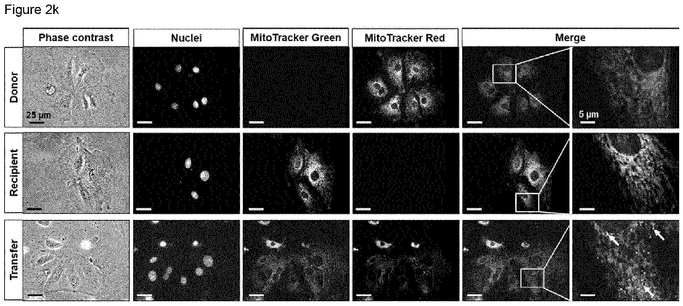

**Figure 2****: Tubular expression of DbpA inversely correlates with the mitochondrial oxygen consumption rate and glycolysis.** (A) Total RNA from kidneys of healthy wild type and *Ybx₃*^{*-*/*-*} mice aged 9-15 weeks (n=3) were isolated and RNA-sequencing was performed. **(B)** Whole RNA-seq data (19,159 transcripts) were summarized in a Volcano plot of differentially expressed genes of healthy kidneys form *Ybx3*^{+/+} versus *Ybx3*^{-/*-*} mice. The most significantly upregulated genes in *Ybx3*^{-/-} mice are shown in red, whereas blue represent the significantly downregulated genes compared to wild type kidneys. Black dots are genes that were differentially expressed below the cutoff values of log-fold change (logFC) and -log₁₀ of the p-value. Significantly regulated transcripts were those with a logFC ≤ ± 1 and -log₁₀ (p-value) >1.3 (dotted lines). **(C)** Pathway analysis of upregulated genes in *Ybx3*^{*-*/*-*} kidneys revealed a strong connection to metabolic pathways and processes. **(D)** The most upregulated genes of these pathways were listed according to their expression level (logFC). Dark red indicates the strongest expression in kidneys from *Ybx3*^{-/-} mice compared to wild type animals. **(E)** Oxygen consumption rates (OCR) of TECs from *Ybx3*^{+/+}*, Ybx3*^{+/-} and *Ybx3*^{-/-} mice were quantified by means of the Seahorse XF Cell Mito Stress Test. OCR were measured under basal level and following addition of 1 µM oligomycin, 1 µM carbonyl cyanide-p-trifluoromethoxyphenylhydrazone (FCCP) and 0.5 µM rotenone + antimycin A (Rot+AA). Genetic deletion of *Ybx3* led to elevated basal and stimulated OCR (n=4, kidneys of three mice were pooled for each experiment). **(F)** Individual parameters for basal respiration, ATP production, proton leakage, maximal respiration rate, spare respiratory capacity and non-mitochondrial oxygen consumption were calculated based on the mitochondrial respiration profiles. All parameters were increased in cells with *Ybx3*^{+/-} or *Ybx3*^{-/*-*} genetic background compared to wild type cells, except for ATP production. **(G)** Enhanced glycolytic activity of *Ybx3*^{-/-} cells was revealed by the detection of extracellular acidification rates (ECAR) of TECs from *Ybx3*^{+/+}*, Ybx3*^{+/-} and *Ybx3*^{*-*/*-*} mice, quantified by means of the Seahorse XF Glycolysis Stress Test. ECAR were measured under basal level and following addition of 10 mM glucose, 1 µM oligomycin and 50 mM of the glucose analog 2-deoxy-glucose (2-DG). Basal ECAR levels were similar in TECs from *Ybx3*^{+/+}*, Ybx3*^{+/-} and *Ybx3*^{*-*/*-*} mice, whereas the addition of glucose led to an increased glycolysis in cells with a genetic deletion of *Ybx3* (n=3, kidneys of three mice were pooled for each experiment). **(H)** Individual parameters for glycolysis, glycolytic capacity, glycolytic reserve and non-glycolytic acidification were calculated based on the Glycolysis Stress Test profile. Glycolysis and glycolytic capacity were increased in *Ybx3*^{*-*/-} cells compared to wild type cells. **(I)** These data were confirmed by the detection of 2-(N-(7-Nitrobenz-2-Oxa-1,3-Diazol-4-yl)Amino)-2-Desoxyglucose (2-NBDG), a fluorescently-labeled deoxyglucose analog, as a measurement of glucose uptake by cultured TECs (n=3-4, kidneys of three mice were pooled for each experiment). **(J)** Mitochondria were isolated from TECs derived from *Ybx3*^{-/-} mice using mitochondria fractionation. Isolated mitochondria were transferred into *Ybx3*^{+/+} TECs and OCR were quantified by means of Seahorse XF Cell Mito Stress Test. Mitochondrial transfers were able to increase basal and stimulated OCR of wild type TECs. **(K)** Successful mitochondrial transfers were confirmed by live cell imaging. Mitochondria of donor TECs were stained with MitoTracker Red CMXRos and mitochondria of recipient cells were stained with MitoTracker Green before the transfer of mitochondria. Yellow dots indicated a successful transfer of donor mitochondria into recipient cells via centrifugation. Scale bars, 25 µm or 5 µm. Data were represented as mean values. All results were confirmed in triplicates by at least two independent experiments. One-way ANOVA followed by Tukey post-test was used for three group comparisons with ^{∗}p<0.05, ^{∗∗}p<0.01.

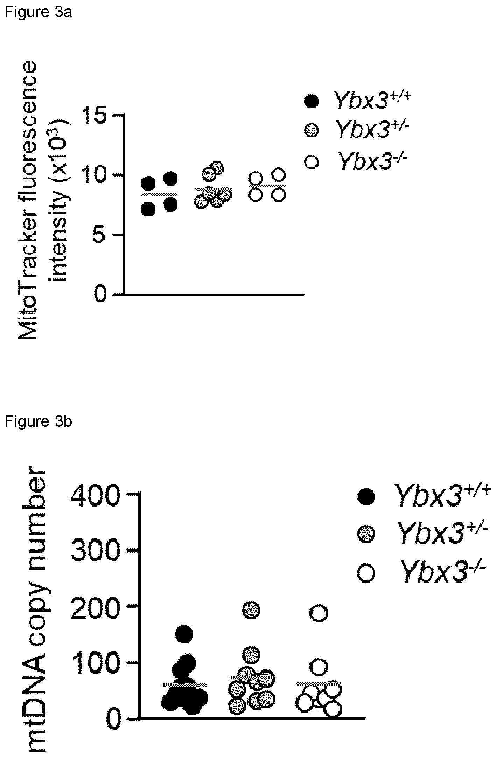

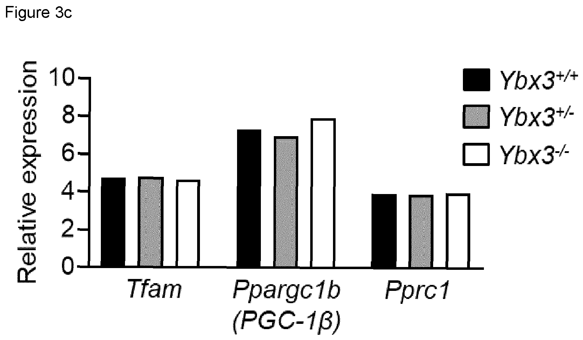

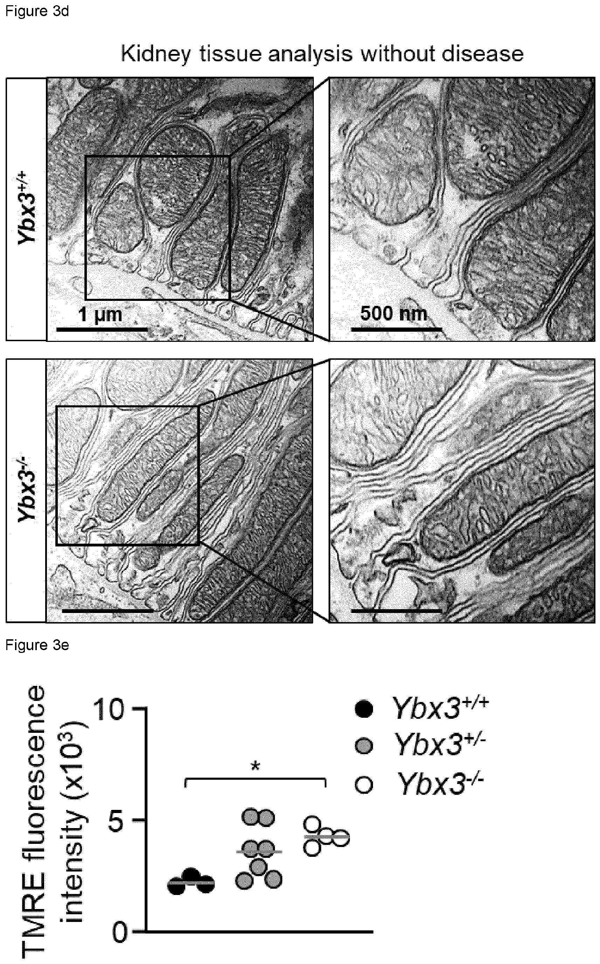

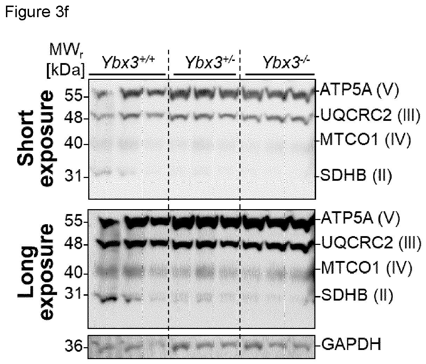

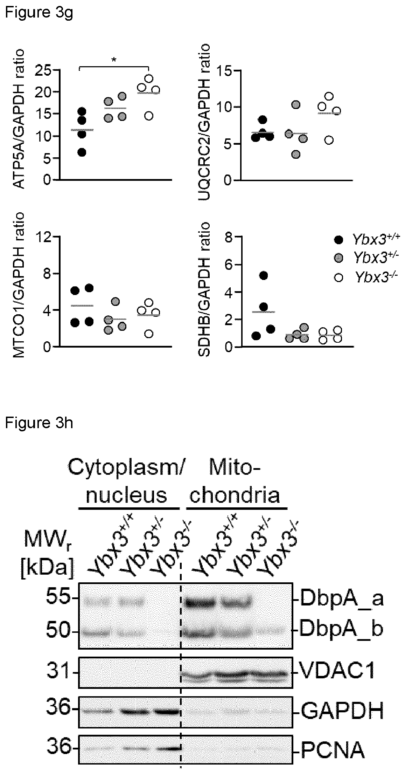

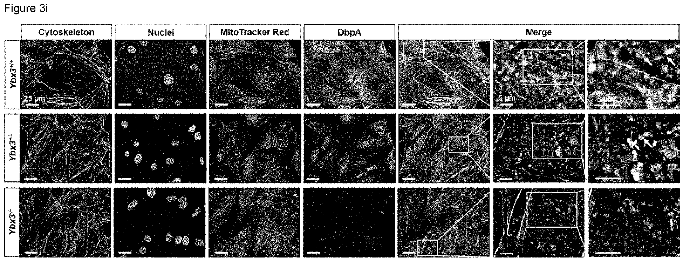

**Figure 3****: DbpA co-localizes with mitochondrial marker proteins.** (A) Mitochondrial mass was quantified by MitoTracker fluorescence intensity of adherent cells (n=4-6). Each individual measurement corresponds to samples with 3 animals pooled. **(B)** Mitochondrial numbers were calculated by mitochondrial DNA (mtDNA) copy numbers that were normalized to nuclear DNA content (n=8-12). Mitochondrial mass and copy numbers were equal in all strains. **(C)** Relative abundance of transcripts involved in mitochondrial gene expression, genome replication and biogenesis was similar between the genotypes, as determined by RNA-seq. **(D)** Integrity of mitochondrial outer and inner membrane was confirmed by transmission electron microscopy of kidney sections. Scale bars, 1 µm or 500 nm. **(E)** Mitochondrial membrane potentials quantified by tetramethylrhodamine ethyl ester (TMRE) assay (n=3-7, kidneys of three mice were pooled for each experiment) were increased in TECs derived from *Ybx3*^{*-*/*-*} mice. **(F)** Representative Western blots from TECs with *Ybx3*^{+/+}*, Ybx3*^{+/-} or *Ybx3*^{-/-} genetic background revealed differential expressions of proteins related to the electron transport chain (ETC). **(G)** Quantification of the Western blots demonstrated enhanced expression of ATP5A (ETC complex V) and UQCRC2 (ETC complex III) in cells with *Ybx3*^{*-*/*-*} genetic background. In contrast the expression of MTCO1 (ETC complex IV) and SDHB (ETC complex II) were increased in wild type cells. **(H)** Following subcellular fractionation into cytoplasmic and mitochondrial proteins and Western blotting for DbpA (anti-C-terminal antibody) as well as VDAC1, GAPDH and PCNA was performed. Purity of fractionation was ascertained by GAPDH (cytosol), PCNA (nucleus) and VDAC1 (mitochondria) co-determinations. Prominent bands for DbpA at 50 and 55 kDa were detected in the mitochondrial fractions. The lysates with *Ybx3*^{-/-} cells yielded a complete absence of DbpA in cytoplasmic/nuclear extracts whereas a weak band corresponding to DbpA_b was detected in the mitochondrial fraction. **(I)** TECs derived from *Ybx3*^{+/+}*, Ybx3*^{+/-} and *Ybx3*^{*-*/*-*} mice were tested for subcellular DbpA localization (green) following co-staining for DAPI (blue; nuclei), Alexa Fluor 488 Phalloidin (white; cytoskeleton) and MitoTracker Red CMXRos (red; mitochondria). TECs from *Ybx3*^{+/+} mice showed DbpA positive staining in the cytoplasm and nucleus. There was less DbpA positive staining in the cytoplasm and nucleus in *Ybx3*^{+/-} TECs, whereas there is almost no DbpA detectable in TECs isolated from *Ybx3*^{*-*/*-*} mice. The overlay of mitochondrial marker protein with the DbpA fluorochrome was visualized by yellow color and demonstrated a co-localization of DbpA with mitochondria. Scale bars, 25 µm or 5 µm. Data were represented as mean values. All results were confirmed in triplicates by at least two independent experiments. One-way ANOVA followed by Tukey post-test was used for three group comparisons with *p<0.05.

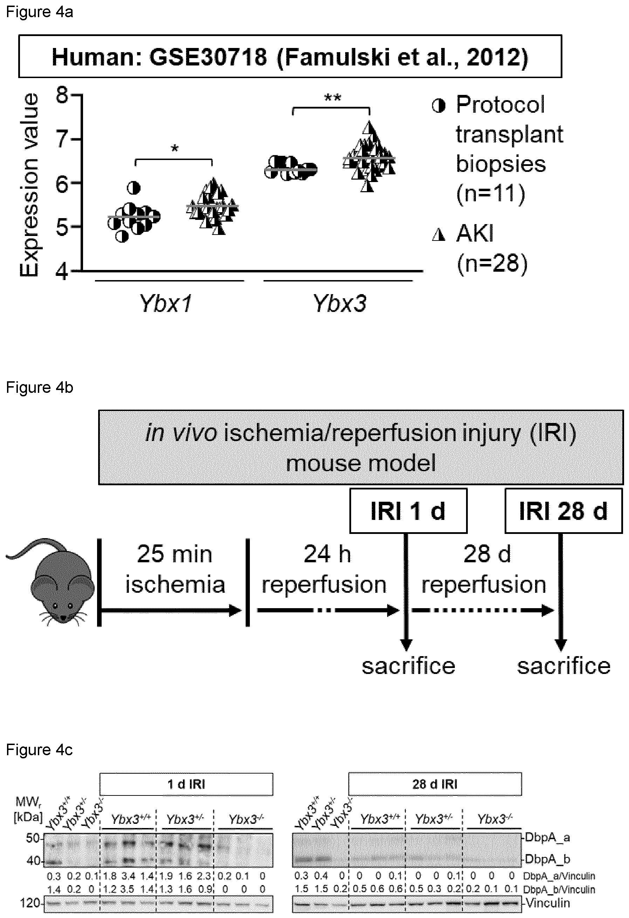

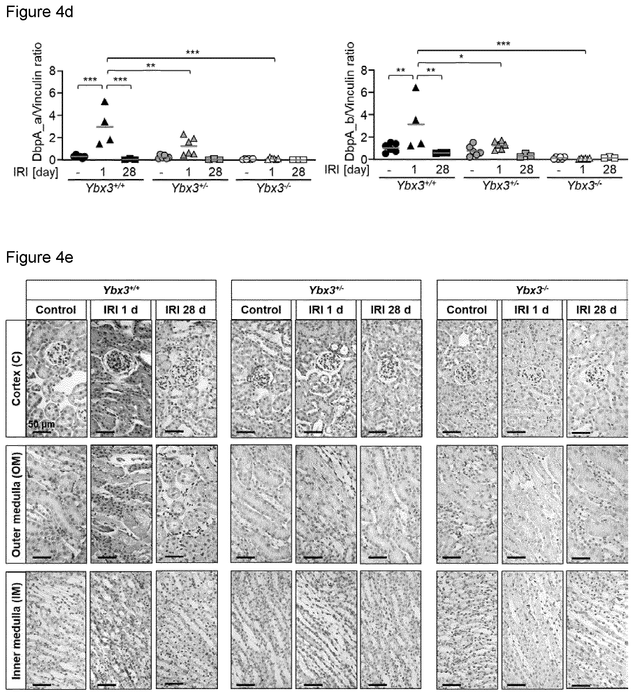

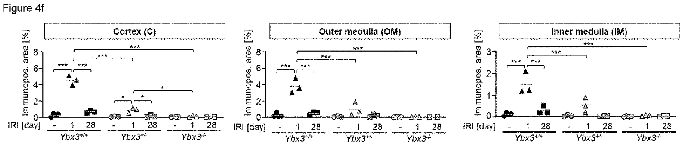

**Figure 4****: *Ybx3* expression is upregulated following acute IRI.**
   **(A)** The gene expression dataset GSE30718 was used for microarray data analysis of human samples from AKI biopsies (n=28) and protocol transplant biopsies (n=11). AKI leads to an increase of *Ybx1* and *Ybx3* expression. **(B)** *In vivo* model of experimental IRI was performed in mice with different genetic background. **(C-D)** Western blot analyses of kidney lysates by means of a polyclonal anti-C-terminal antibody against DbpA indicated low level expression of DbpA in healthy kidney tissue, which was strongly upregulated 1 d following IRI. Expression levels returned to basal levels within 28 d. **(D and E)** Immunohistochemistry of kidney tissues for DbpA expression in mice aged 8-12 weeks (using antibody directed against the C-terminus) demonstrated immunopositive cells in tubular and glomerular structures. 1 d following IRI, a substantial DbpA upregulation in tubular cells was seen in wild type animals, which was less prominent in *Ybx3*^{+/-} and not seen at all in *Ybx3*^{-/-} animals. Scale bars, 50 µm. Data were represented as mean values. All results were confirmed in triplicates by at least two independent experiments. One-way ANOVA followed by Tukey post-test was used for three group comparisons with ^{∗}p<0.05, ^{∗∗}p<0.01, ^{∗∗∗}p<0.

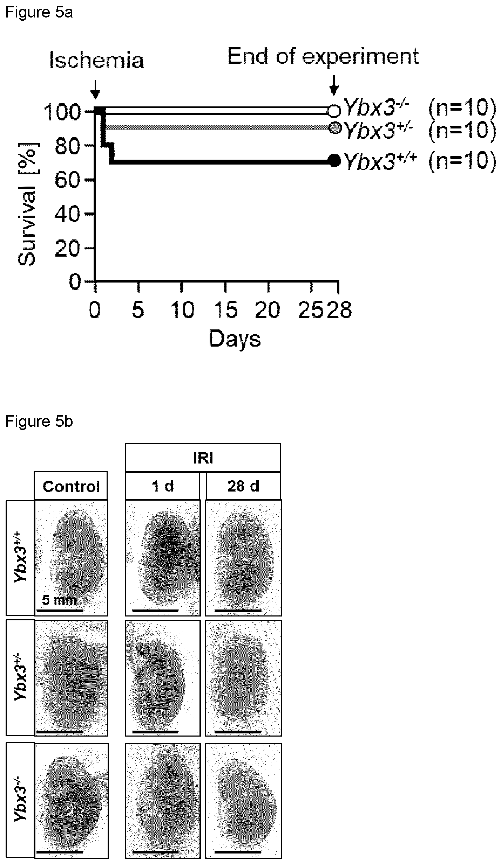

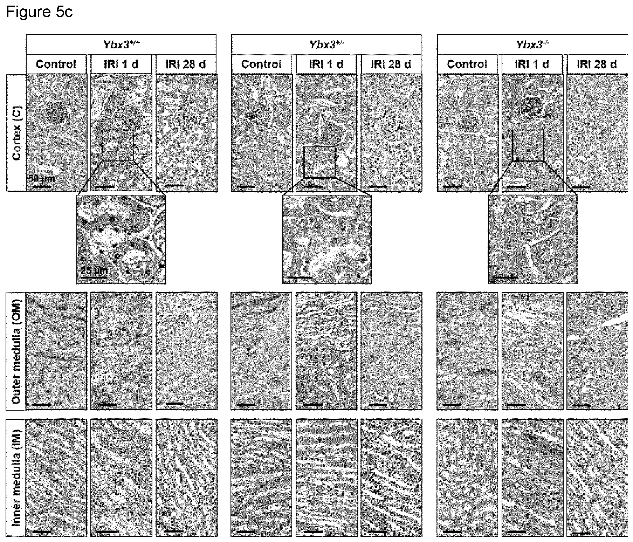

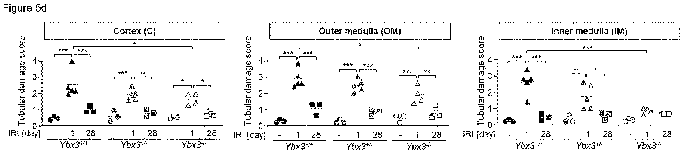

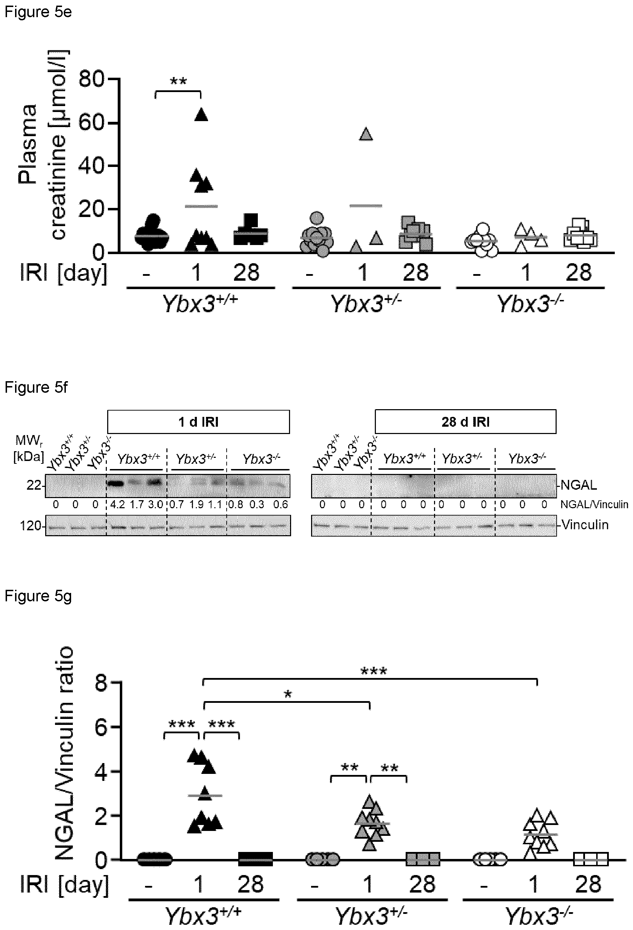

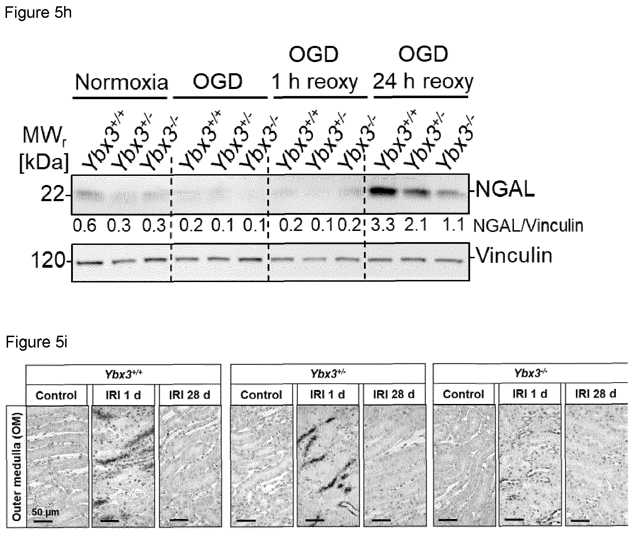

**Figure 5****: DbpA expression confers tubular cell damage.**
   **(A)** Survival analysis over 28 d was performed following ischemia and subsequent reperfusion of the kidneys. Significantly higher mortality rates in wild type than in *Ybx3*^{-/-} animals became apparent (n=10). **(B)** Representative macroscopic images of longitudinal kidney sections obtained following ischemia and 1 d as well as 28 d reperfusion periods. In wild type animals the kidney tissue appeared congested and filled with blood 1 d following IRI although PBS perfusion was performed prior harvesting of the kidneys. Scale bars, 5 mm. **(C)** PAS staining of kidney sections revealed increased tubular cell damage following IRI in wild type animals in cortex, OM and IM. Enlargement of images for IRI 1 d obtained from the cortex visualized nuclear condensation of the majority of tubular cells, that was less abundant in *Ybx3*^{+/-} animals and not present in *Ybx3*^{-/-} animals. Scale bars, 50 µm or 25 µm (quantified in **D**). (**E**) Kidney function assessed by the determination of plasma creatinine levels indicated severe AKI 1 d following IRI in wild type animals, whereas creatinine values were unaltered in *Ybx₃*^{-/-} animals. **(F** and **G)** Kidney damage protein neutrophil gelatinase-associated lipocalin (NGAL) was detected by Western blot analyses of kidney tissue lysates. For *Ybx3*^{*-*/*-*} animals there was no clear upregulation of NGAL 1 d following IRI. In control animals and 28 d following IRI, NGAL was not detectable. **(H)** These data were confirmed by Western blot analysis of TECs obtained from *Ybx3*^{+/+}*, Ybx3*^{+/-} and *Ybx3*^{*-*/*-*} mice, where NGAL expression was increased 24 h following oxygen and glucose deprivation (OGD). **(I)** Immunohistochemistry of kidney tissue for NGAL expression revealed increased levels in the OM of *Ybx3*^{+/+} mice 1 d following IRI compared to *Ybx3*^{*-*/*-*} animals. Scale bars, 50 µm. Data were represented as mean values. All results were confirmed in triplicates by at least two independent experiments. One-way ANOVA followed by Tukey post-test was used for three group comparisons with ^{∗}p<0.05, ^{∗∗}p<0.01, ^{∗∗∗}p<0.001.

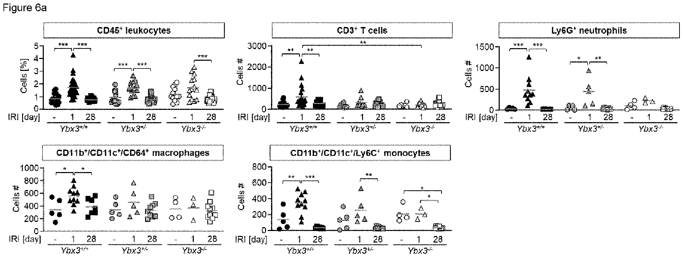

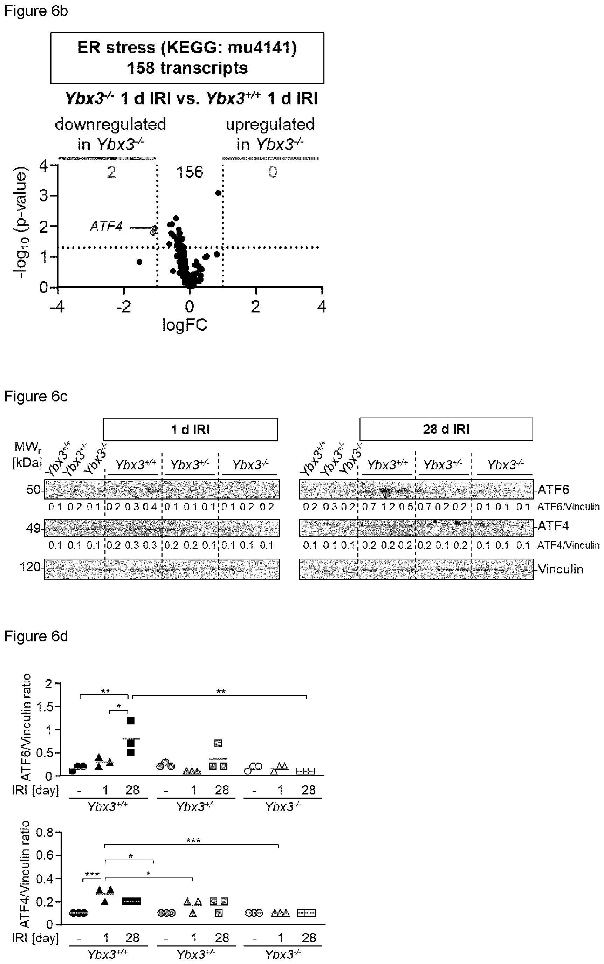

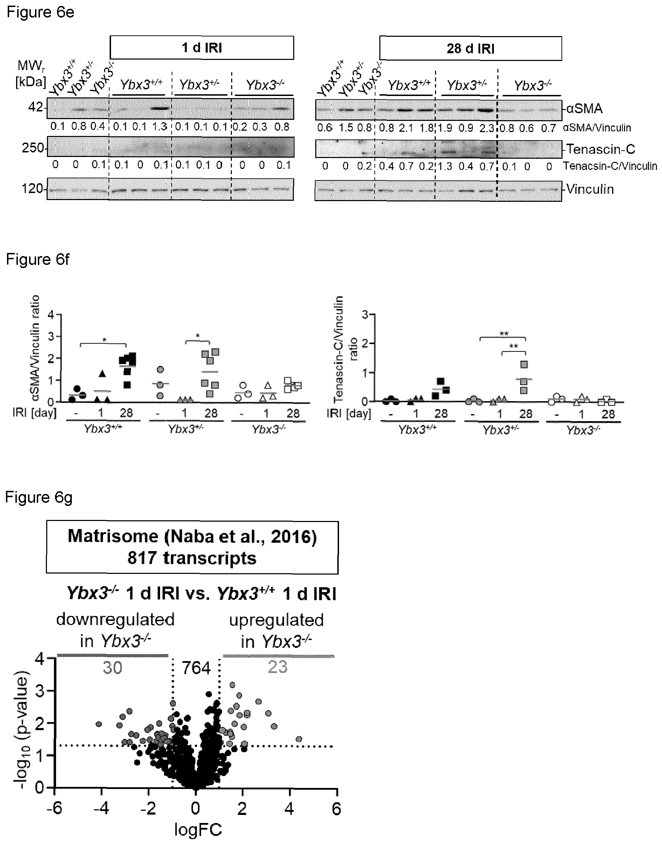

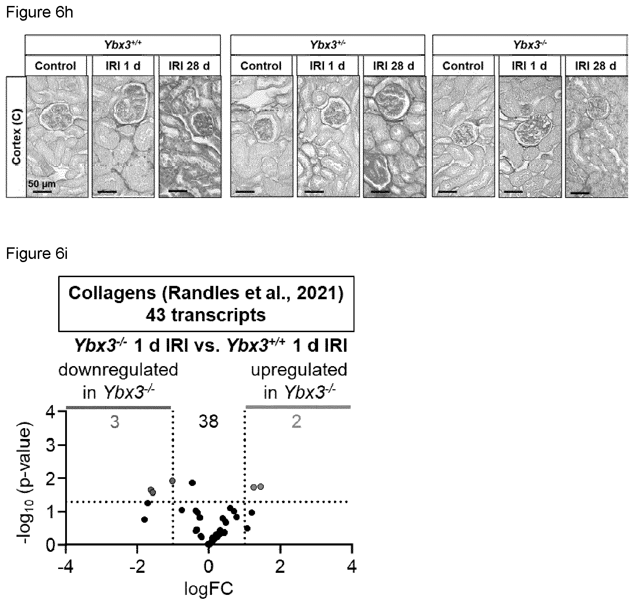

**Figure 6****: *Ybx3*^{*-*/*-*} animals do not develop an inflammatory and fibrotic response.**
   **(A)** Infiltrating immune cells quantified by flow cytometry following kidney tissue disintegration revealed a similar influx of CD45⁺ leukocytes in the kidneys of all genotypes 1 d following IRI. Subanalysis of CD3⁺ T cells, Ly6G⁺ neutrophils, CD11b⁺/CD11c⁺/CD64⁺ macrophages and CD11b⁺/CD11c⁺/Ly6C⁺ monocytes revealed significantly less immune cell infiltration in kidneys of *Ybx3*^{*-*/*-*} animals 1 d following IRI. 28 d following IRI the numbers of immune cells returned to basal levels. **(B)** Transcript analysis by RNA-seq of the KEGG: mu4141 dataset indicating downregulation of *ATF4* and *PPP1R15A* (blue) in kidneys of *Ybx3*^{*-*/-} mice compared to wild type. 156 transcripts were not regulated (black). **(C** and **D)** The expression of ER stress marker proteins transcription factor 6 (ATF6) and activating transcription factor 4 (ATF4) were significantly increased in wild type animals following IRI. Whereas activation of ER stress did not occur in *Ybx3*^{*-*/*-*} animals following IRI. **(E** and **F)** Kidney lysates from animals 1 d and 28 d following IRI quantified the expression of alpha smooth muscle actin (αSMA) and Tenascin-C as markers for fibrosis. αSMA and Tenascin-C were significantly less abundant in *Ybx3*^{-/-} animals 28 d following ischemia. **(G)** In addition, RNA-seq data demonstrated an increased number of downregulated genes (blue) related to matrisome (according to Naba et al., 2016) in the kidney of *Ybx3*^{*-*/*-*} mice compared to wild type. Red represents upregulated transcripts in *Ybx3*^{*-*/*-*} mice. 764 transcripts were not differentially expressed (black) between the genotypes. **(H)** Picro sirius red staining of kidney sections at time points 1 d and 28 d following ischemia visualized fibrosis as reddish color. A prominent tubulointerstitial and glomerular collagen deposition 28 d following IRI was seen for the cortex in animals with *Ybx3*^{+/+} and *Ybx3*^{+/-} genetic background. In contrast, *Ybx3*^{*-*/*-*} animals exhibited minor tubulointerstitial and glomerular fibrosis and collagen deposition. Scale bars, 50 µm. **(I)** Moreover, RNA-seq data demonstrated an increased number of downregulated genes (blue) related to collagens (according to Randles et al., 2021) in the kidney of *Ybx3*^{-/-} mice compared to wild type. Red represented upregulated transcripts in *Ybx3*^{*-*/*-*} mice, whereas 38 transcripts were not differentially expressed (black). Data were represented as mean values. All results were confirmed in triplicates by at least two independent experiments. One-way ANOVA followed by Tukey post-test was used for three group comparisons with *p<0.05, **p<0.01, ***p<0.001.

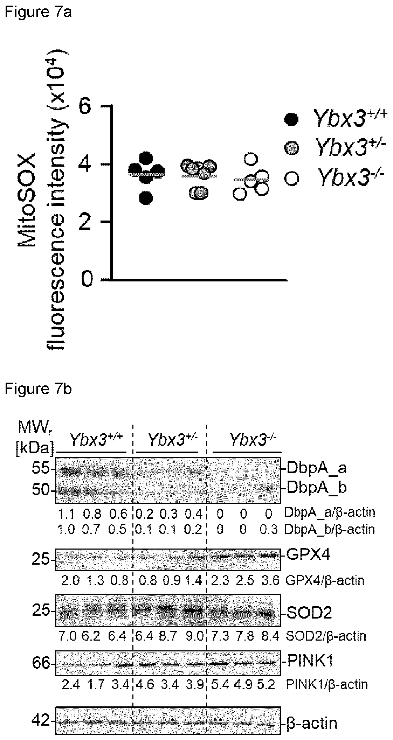

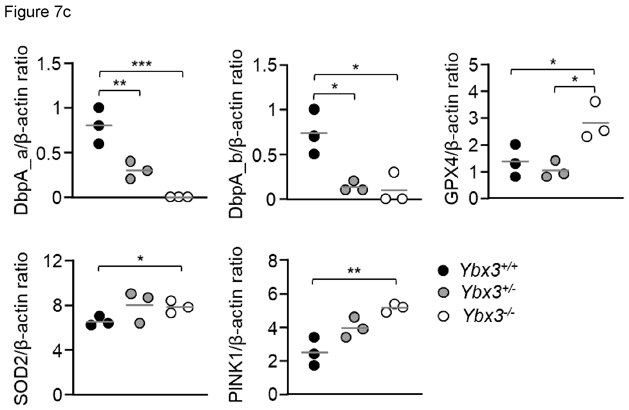

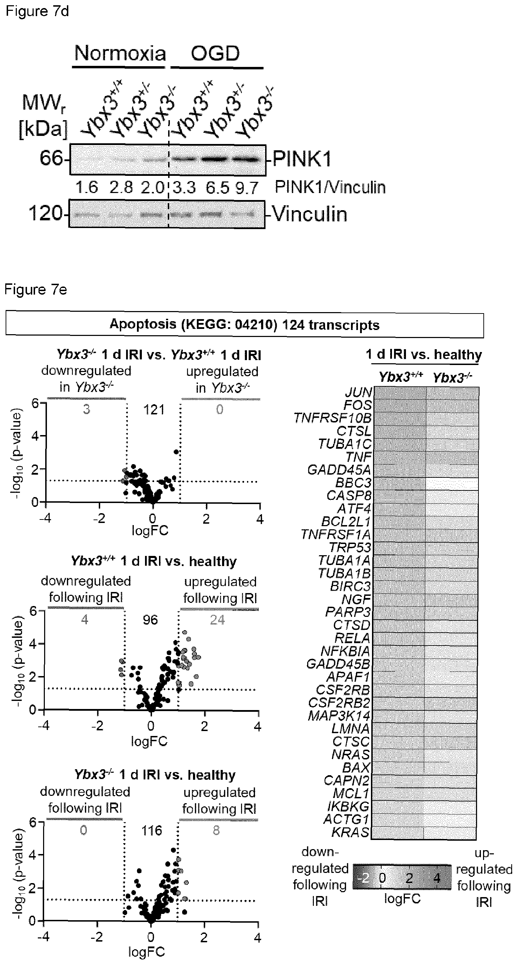

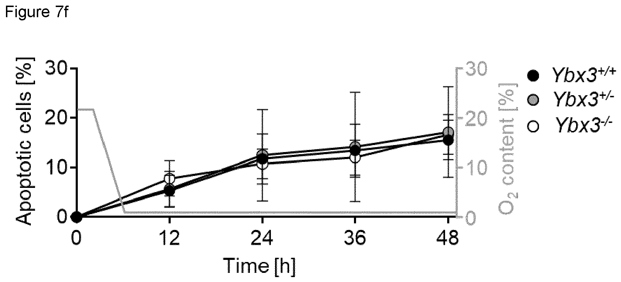

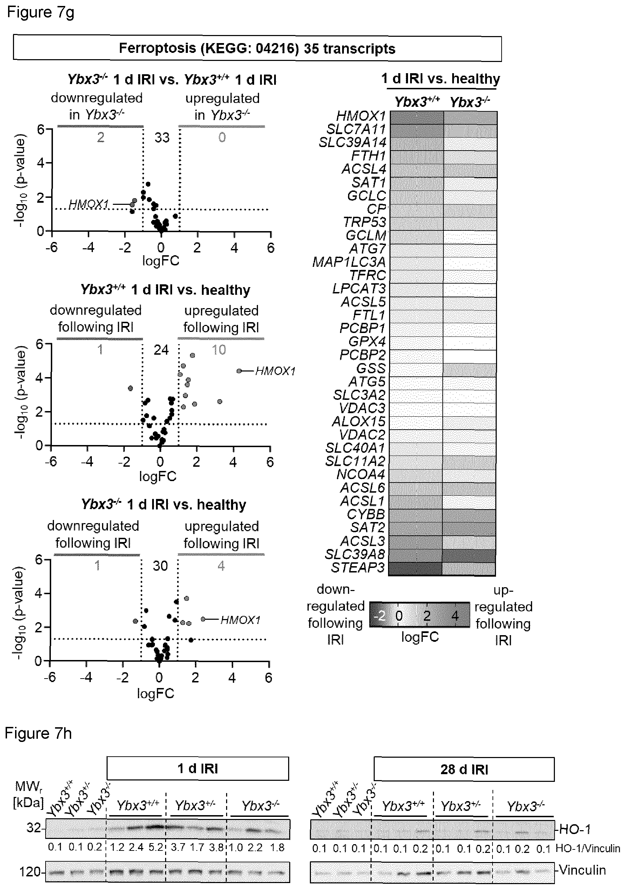

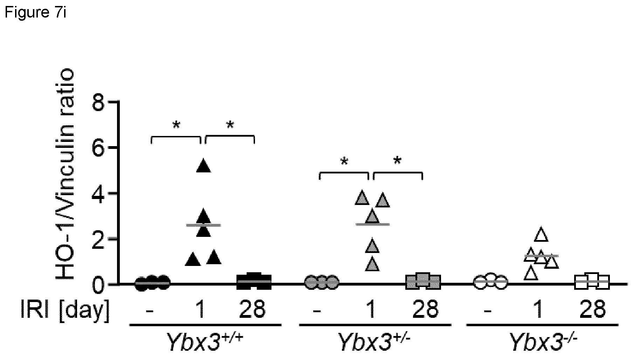

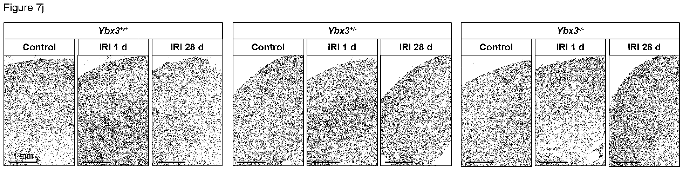

**Figure 7****. Increased antioxidant activity provides the basis for the protective mechanism.**
   (A) Basal mitochondrial superoxide (MitoSOX) levels were similar in TECs under normoxic conditions (n=5-7, kidneys of three mice were pooled for each experiment). **(B)** Western blot analyses of TECs obtained from *Ybx3*^{+/+}*, Ybx3*^{+/-} and *Ybx3*^{-/-} mice were performed to analyze the expression of antioxidant proteins (GPX4 and SOD2) and mitophagy markers (PINK1). **(C)** Increased expression levels of these proteins were detected in *Ybx3*^{*-*/*-*} tubular cells compared to wild type. **(D)** Furthermore, PINK1 expression was increased following OGD in *Ybx3*^{*-*/*-*} TECs. **(E)** RNA-seq data revealed no significant differences in the regulation of apoptotic genes (KEGG: 04210) in kidneys 1 d following IRI in wild type versus *Ybx3*^{*-*/*-*} mice. Blue dots represent downregulated genes. Red shows upregulation and black not differentially expressed genes. Top 35 differential expressed transcripts of wild type animals were shown in the heat map. **(F)** These data were confirmed by live cell imaging of TECs following OGD. No differences in the number of apoptotic cells were revealed. After Hoechst and PI staining of TECs, they were analyzed under reduced oxygen and nutrition levels for 48 h. PI positive cells were detected as death cells and mean relative healthy nuclei were calculated over 48 h. O₂ content in the hypoxic chamber was reduced from 21% to 1% after 2 h until the end of the experiment. **(G)** Analysis of ferroptosis related transcripts (KEGG: 04216) revealed enhanced *HMOX1* expression 1 d following IRI in wild type animals and less upregulation of ferroptosis-related genes in *Ybx₃*^{-/-} animals. Blue represents downregulation, red upregulation and black not differentially expressed genes. **(H** and **I)** These data were confirmed by Western blot analysis, where HO-1 was increased expressed in wild type animals 1 d following IRI. **(J)** 4HNE staining of kidney sections showed increased ferroptosis in wild type and *Ybx3*^{*+*/*-*} animals especially in the outer medulla compared to *Ybx3*^{-/-} animals 1 d following IRI. 28 d following ischemia, positive staining reached control levels. Data were represented as mean values. All results were confirmed in triplicates by at least two independent experiments. One-way ANOVA followed by Tukey post-test was used for three group comparisons with ^{∗}p<0.05, ^{∗∗}p<0.01, ^{∗∗∗}p<0.001.
**Figure 8****: Suitable anti-DbpA antibodies.**
   Specific antibody binding regions against isoform 1 (DbpA a) and isoform 2 (DbpA_b) are located within the N-terminal region (denoted Royal, DbpA (Cheng) and Waldo) or the C-terminal region (denoted: Curacao and Hugo, respectively). Hugo is specific for isoform 1. The designation of the antibodies outlined includes the laboratory ID number and laboratory denomination as well as the amino acid sequence within the DbpA protein.

### EXAMPLES

The examples given below are for illustrative purposes only and do not limit the invention described above in any way.

### MATERIALS & METHODS

### Experimental model and subject details

### Mice

Animal studies were performed with genetically modified mice on a C57BL/6N background. Equal numbers of males and females were analyzed. Animals were maintained according to the FELASA guidelines (Federation of European Laboratory Animal Science Association) under specific pathogen-free conditions. All procedures were performed in accordance with the German National Guidelines for the use of experimental animals and approved by the state of Saxony-Anhalt (AZ UniMD 42502-2-1634 UniMD).

### Method details

### Renal ischemia/reperfusion injury (IRI) model

IRI was performed with gender matched 9 to 14 weeks old mice. Renal arteries and veins were occluded bilaterally with surgical clamps for 25 min. The clamps were removed and the kidneys were examined to ensure renal perfusion. All animals received a subcutaneous analgesic at the end of the procedure. Mice were placed in a temperature-controlled environment during the recovery phase and returned to their cages with free access to food and water. In control animal no ischemia was performed. Animals were sacrificed 24 h (1 d) or 28 days (28 d) following renal IRI to obtain blood and tissue samples.

### Blood and plasma analyses

Blood samplings by heart puncture were subjected to a complete blood cell count (ADVIA 120, Bayer Diagnostics Munich). Renal function was assessed by measuring plasma creatinine using an enzymatic assay and blood urea nitrogen (BUN) with a kinetic assay with urease (Roche Diagnostics, Cobas c501 module) according to the manufacturer's instructions.

### Urine measurements

Urine samples from mice were collected individually in metabolic cages (Tecniplast). The metabolic parameters albumin and pH were determined using NobiStrip U10 test strips (Hitado) according to the manufacturer's instructions. Urine osmolality was measured according to the manufacturer's instructions (Roche Diagnostics, Cobas c501 module). Protein content was determined using the Quick Start Bradford protein assay (Bio-Rad).

### Transcutaneous measurement of glomerular filtration rate (GFR)

GFR was measured in 3-months old mice by the transcutaneous clearance of fluorescein isothiocyanate (FITC)-sinistrin using a NIC-kidney device (Mannheim Pharma & Diagnostics) as previously described (Schock-Kusch et al., 2009).

### Tissue lysate preparation and Western blot analysis

Tissues were mechanically homogenized in RIPA buffer containing complete protease inhibitor cocktail and PhosStop (Sigma-Aldrich). Protein content was determined using the DC Protein Assay Kit II (Bio-Rad). Denatured protein samples were separated using 10% SDS-PAGE and blotting on nitrocellulose membranes. Membranes were blocked with 5% dry milk in TBS/Tween and incubated with primary antibodies diluted overnight at 4°C, followed by incubation with HRP-conjugated secondary antibody for 1 h. Pierce ECL Western Blotting-Substrat (Thermo Fisher Scientific) was used and emitted light measured using an Intas imaging system (Intas Science Imaging Instruments). The antibodies used for Western blot are listed in **Table 1** below.

**Table 1: Antibodies used for Western blot.**

| **Antibody** | **Species** | **Company** | **Catalog nr.** | **Dilution** |
|---|---|---|---|---|
| αSMA | Rabbit | Abcam | ab5694 | 1:1000 |
| β-actin | Mouse | Sigma | A1978 | 1:1000 |
| AE1 | Rabbit | Merck Milipore | AB3500P | 1:500 |
| Akt | Rabbit | Cell Signaling Technology | 9272 | 1:1000 |
| AMPKα | Rabbit | Cell Signaling Technology | 2532 | 1:1000 |
| Aqp1 | Rabbit | Alpha diagnostic international | AQP11 | 1:1000 |
| Aqp2 | Rabbit | Abcam | Ab199975 | 1:500 |
| ATF4 | Rabbit | Cell Signaling Technology | 11815 | 1:500 |
| ATF6 | Rabbit | Cell Signaling Technology | 65880 | 1:500 |
| Atp6v1b1 | Rabbit | Invitrogen | PA5-56878 | 1:500 |
| CAII | Rabbit | Abcam | ab191343 | 1:1000 |
| DbpA C-terminal | Rabbit | Eurogentec | ED19016 | 1:1000 |
| ERK1/2 | Mouse | Cell Signaling Technology | 4696 | 1:1000 |
| GAPDH | Goat | GenScript Biotech | A00191-40 | 1:1000 |
| GPX4 | Mouse | Santa Cruz Biotechnology | sc-166120 | 1:500 |
| HIF1α | Rabbit | Cell Signaling Technology | 14179 | 1:1000 |
| JNK | Rabbit | Cell Signaling Technology | 9252 | 1:1000 |
| NGAL | Goat | R&D Systems | AF1757 | 1:500 |
| p38 | Rabbit | Cell Signaling Technology | 9212 | 1:1000 |
| PCNA | Mouse | Santa Cruz Biotechnology | sc-56 | 1:1000 |
| Phospho-AMPKα (T¹⁷²) | Rabbit | Cell Signaling Technology | 2535 | 1:1000 |
| Phospho-Akt (S⁴⁷³) | Rabbit | Cell Signaling Technology | 4060 | 1:1000 |
| Phospho-ERK1/2 (T²⁰²/Y²⁰⁴) | Rabbit | Cell Signaling Technology | 4370 | 1:1000 |
| Phospho-JNK (T¹⁸³/Y¹⁸⁵) | Rabbit | Cell Signaling Technology | 9251 | 1:1000 |
| Phospho-p38 (T¹⁸⁰/Y¹⁸²) | Rabbit | Cell Signaling Technology | 9211 | 1:1000 |
| PINK1 | Mouse | Santa Cruz Biotechnology | sc-517353 | 1:1000 |
| SOD2 | Mouse | Santa Cruz Biotechnology | sc-137254 | 1:500 |
| Tenascin-C | Rabbit | Abcam | ab108930 | 1:1000 |
| Total OXPHOS Rodent WB Antibody Cocktail | Mouse | Abcam | ab110413 | 1:250 |
| VDAC1 | Rabbit | Abcam | ab15895 | 1:1000 |
| Vinculin | Mouse | Santa Cruz Biotechnology | sc-59803 | 1:1000 |
| Goat anti-mouse IgG HIZP | Goat | Southern Biotech | 1031-05 | 1:5000 |
| Goat anti-rabbit IgG HIZP | Goat | Southern Biotech | 4050-05 | 1:5000 |
| Donkey anti-goat IgG HIZP | Donkey | Jackson ImmunoResearch | 705-035-147 | 1:5000 |

The NuPAGE Western blot system from Invitrogen (Thermo Fisher Scientific) was used to detect cold shock protein DbpA according to the manufacturer's instructions.

### Histology

Formalin-fixed, paraffin-embedded 2-4 µm tissue sections were deparaffinized in xylene, dehydrated and boiled in sodium citrate buffer for antigen unmasking. Endogenous peroxidase was inactivated by incubation in 3% hydrogen peroxide. The sections were incubated overnight with primary antibodies. Afterwards, the sections were incubated with peroxidase-labeled secondary antibodies for 45 min. The sections were developed with diaminobenzidine substrate and counterstained with hematoxylin solution to visualize nuclei. Dehydration was achieved by an ascending alcohol series. Embedding was performed with ROTI^{®} Mount (Carl Roth). For immunofluorescence staining, coverslips were mounted using ProLong Gold Antifade mountant with DAPI (Thermo Fisher Scientific). For Periodic acid-Schiff (PAS) staining, tissue sections were stained in periodic acid solution and incubated in Schiff's reagent. For Picro Sirius Red staining, the sections were incubated with 0.1% Sirius Red in saturated picric acid and were destained with 0.01 N hydrochloric acid. Tissue sections were imaged with a Leica DM6000B microscope (Leica Microsystems) and ImageJ for image acquisition. The antibodies used for immunohistochemistry are listed in **Table 2** below. For transmission electron microscopy, fixed kidneys were post-fixed with osmium tetroxide and embedded in epoxy resin. Ultra-thin sections (70 nm) were stained with uranyl acetate.

**Table 2: Primary and secondary antibodies used for immunohistochemistry and immunofluorescence staining**

| **Antibody** | **Species** | **Company** | **Catalog nr.** | **Dilution** |
|---|---|---|---|---|
| 4HNE | Rabbit | Abcam | ab46545 | 1:500 |
| Atp6v1b1 | Rabbit | Invitrogen | PA5-56878 | 1:50 |
| Aqp2 | Mouse | Santa Cruz Biotechnology | sc-515770 | 1:50 |
| Calbindin D-28K | Chicken | Novus Biologicals | NBP2-50028 | 1:100 |
| DbpA | Rabbit | Eurogentec | ED15001 | 1:500 |
| NGAL | Rabbit | Boster Immunoleader | PB9609 | 1:500 |
| Goat anti-rabbit IgG HRP | Goat | DAKO/AgilentTechnologi es | P0448 | 1:200 |
| Goat anti- mouse IgG TRITC | Goat | Jackson Immno Research | 115-026-003 | 1:100 |
| Goat anti-rabbit IgG Alexa Fluor 488 | Goat | Thermo Fisher Scientific | A11070 | 1:100 |
| Goat anti-chicken IgY Alexa Fluor 647 | Goat | Thermo Fisher Scientific | A-21449 | 1:100 |

### Flow cytometry

Single cell suspensions of kidney tissue samples were labeled with antibodies directed against surface receptors/proteins. Fixation and cell membrane permeabilization were performed using the FOXP3 Fix/Perm buffer set (BioLegend). Flow cytometry was performed with a FACS Canto II (BD Bioscience). Data acquisition and analysis were performed using FlowJo software. Antibodies used for flow cytometry are listed in **Table 3** below.

**Table 3: Antibodies used for flow cytometry**

| **Antibody** | **Fluorochrome** | **Company** | **Catalog nr.** | **Dilution** |
|---|---|---|---|---|
| CD3 | APC | eBioscience | 17-0031 | 1:100 |
| CD11c | APC | Biozol | 117309 | 1:200 |
| CD11b | APC-Cy7 | BioLegend | 101226 | 1:200 |
| CD45 | BV510 | BioLegend | 103138 | 1:500 |
| CD64 | PE | BioLegend | 139304 | 1:100 |
| Ly6C | BV421 | BioLegend | 128032 | 1:200 |
| Ly6G | PE-Cy7 | BioLegend | 127618 | 1:1000 |
| MHC-II | FITC | BioLegend | 107606 | 1:200 |

### Isolation of total RNA and quantitative PCR

Total RNA was extracted from kidney tissue samples using TriFast reagent (Peqlab) according to the manufacturer's instructions. RNA was reverse transcribed using the Transcriptor High Fidelity cDNA kit (Sigma-Aldrich). PCR was performed using the PCR Mastermix kit (Qiagen). Products were separated on 2% agarose gels. Primers are listed in **Table 4** below.

**Table 4: Primers used for PCR.**

| **Allele** | **Primer name** | **SEQ ID NO:** | **Primer sequence** |
|---|---|---|---|
| *β-actin* | fwd | 2 | AGAGAGGTATCCTGACCCTGAAGT |
| | rev | 3 | CACGCAGCTCATTGTAGAAGGTGT |
| *Aqp2* | fwd | 4 | ATGTGGGAACTCCGGTCCATA |
| | rev | 5 | ACGGCAATCTGGAGCACAG |
| *Atp6v1b1* | fwd | 6 | TGCTCTACCTGGAGTTCCTGCAGAAGTTTGAGAAG |
| | rev | 7 | TCATGCTCTGCGGAATGCGCTTCAGCATCTCTTTC |
| *B2m* | fwd | 8 | AGCAAAGAGGCCTAATTGAAGTC |
| | rev | 9 | GAAGTAGCCACAGGGTTGGG |
| *mt1* | fwd | 10 | TGAACGGCTAAACGAGGGTC |
| | rev | 11 | AGCTCCATAGGGTCTTCTCGT |
| *mt2* | fwd | 12 | CAGTCCCCTCCCTAGGACTT |
| | rev | 13 | ACCCTGGTCGGTTTGATGTT |
| *mt3* | fwd | 14 | TAATCGCACATGGCCTCACA |
| | rev | 15 | GAAGTCCTCGGGCCATGATT |
| *Tuba1a* | fwd | 16 | CCGCGAAGCAGCAACCAT |
| | rev | 17 | CCAGGTCTACGAACACTGCC |
| *Ybx3* | fwd | 18 | CAGACTCAGAAAATGAAGGGCA |
| | rev | 19 | AGATGGGCAAGATTCTCAAGTC |

### Transcriptome analysis using RNA-seq and bioinformatic analysis

RNA was quantified using a NanoDrop-1000 spectrophotometer (Thermo Fisher Scientific). The quality and integrity of total RNA was monitored using an Agilent Technologies 2100 Bioanalyzer (Agilent Technologies). The RNA-sequencing library was prepared from 500 ng of total RNA using the Dynabeads mRNA DIRECT Micro Purification Kit (Thermo Fisher Scientific). For mRNA purification the NEBNext Ultra II Directional RNA Library Prep Kit (New England BioLabs) was used according to the manufacture's protocol. Libraries were sequenced on Illumina NovaSeq 6000 Sequencing System using NovaSeq 6000 S1 Reagent Kit (Illumina) with an average of 3×10⁷ reads per RNA sample. Each FASTQ file receives a quality report generated by the FASTQC tool. Before alignment to the reference genome, each sequence in the raw FASTQ files was trimmed for base call quality and sequencing adapter contamination using the Trim Galore! wrapper tool. Reads shorter than 20 bp were removed from the FASTQ file. Trimmed reads were aligned to the reference genome using the open-source short read aligner STAR (https://code.google.com/p/rna-star/) with the settings according to log file. Feature counts were determined using the R package "Rsubread". Only genes that had a value greater than 5 at least twice in all samples were considered for further analysis. Gene annotation was performed using the R package "bioMaRt". Before starting the statistical analysis steps, expression data were log2 transform and normalized according to TMM normalization using "edgeR" package. Differential gene expression was calculated using the R package "edgeR". Each list of differentially expressed genes derived from the respective comparisons was subjected to functional and biochemical pathway analysis using g:Profiler. Enrichment blots were generated in Python using the Matplotlib library and the Spyder programming environment.

### Isolation of primary tubular epithelial cells (TECs) from mice

Kidneys were isolated, mechanically crushed and incubated in digestion buffer containing 1 mg/ml collagenase. Tissue lysates were resuspended in DBPS and passed through cell filters. Tubular cells were resuspended in DMEM supplemented with 10% fetal bovine serum and 50 U/ml penicillin, 50 mg/ml streptomycin, and a hormone mixture (1.25 ng/ml prostaglandin E1, 34 pg/ml triiodothyronine, 50 nM hydrocortisone, and 25 ng/ml epidermal growth factor, insulin-transferrin-selenium) and transferred into a cell culture flask.

### Isolation of primary bone marrow-derived macrophages (BMDMs) from mice

BMDMs were obtained from the femurs of mice. The cleaned bones were crushed several times using a pestle and washed with DPBS. Bone marrow cells were collected after filtration through a cell strainer and centrifuged at 1400 rpm at room temperature for 5 min. The supernatant was decanted and the pellet resuspended in ammonium chloride-potassium buffer to lyse the erythrocytes. After washing, the cells were cultured in DMEM supplemented with 10% fetal bovine serum and 50 U/ml penicillin, 50 mg/ml streptomycin and 10 ng/ml M-CSF overnight in a cell culture plate. The next day, non-adherent cells were cultured for 6 days in cell culture medium with 10 ng/ml M-CSF.

### In vitro hypoxia and reoxygenation (HR) model

To mimic a renal IRI, the *in vitro* oxygen and glucose deprivation (OGD) model was performed. Primary cells were maintained in DPBS (+CaCl₂ +MgCl₂) in a hypoxic atmosphere containing 1% O₂, 94% N₂ and 5% CO₂ for 6 hours. For reoxygenation, cells were again maintained in complete medium and 21% O₂. Control cells were continually treated with 21% O₂.

### Immunofluorescence staining of primary cells on glass slides

Primary cells were seeded onto glass slides. For mitochondrial staining, MitoTracker Red CMXRos (Thermo Fisher Scientific) was used according to the manufacturer's instructions. Cells were fixed with 4% PFA and permeabilized. Primary antibodies against DbpA and fluorescence-labeled secondary antibodies were incubated subsequently (see **Table 1** above). Alexa Fluor 488 phalloidin (Thermo Fisher Scientific) was used to visualize the actin/cytoskeleton. Glass slides were mounted with ProLong Gold Antifade mountant with DAPI (Thermo Fisher Scientific). Leica DM6000B microscope with Leica application suite advanced fluorescence software was used for imaging.

### Mitochondrial assays

The Agilent Seahorse XF (Agilent Technologies) system was used to determine mitochondrial respiration of primary cells. Therefore, cells were transferred to a Seahorse XF96 cell culture microplate (10,000 cells/well) and rested overnight in cell culture medium. The Seahorse XF Cell Mito Stress Test kit, Seahorse XF Glycolysis Stress Test kit and Seahorse XFe96 Analyzer were used according to the manufacturer's instructions. Glucose uptake was measured using 2-(N-(7-Nitrobenz-2-oxa-1,3-diazol-4-yl)Amino)-2-Desoxyglucose (2-NBDG)-Assay (Thermo Fisher Scientific), according to the manufacturer's instructions. MitoSOX Red Mitochondrial Superoxide Indicator (Thermo Fisher Scientific) was used to detect mitochondrial superoxide levels. Adherent cells were stained and MitoSOX fluorescence intensity was measured using a fluorescence plate reader. TMRE (tetramethylrhodamine, ethyl ester)-mitochondrial membrane potential assay kit (Abcam) was used according to the manufacturer's instructions. For determination of mitochondrial mass, primary cells were stained with MitoTracker Red CMXRos (Thermo Fisher Scientific) according to the manufacturer's instructions. To determine mitochondrial copy number, total DNA was isolated from primary cells using the FlexiGene DNA kit (Qiagen). Primers against three different regions of mitochondrial DNA (*mt1, mt2, mt3*)*,* and three regions of genomic DNA (*B2m, Tubala, β-actin*) were used (see **Table 2** above). Quantitative real time PCR was performed using SYBR Green qPCR MasterMix (Thermo Fisher Scientific). The mtDNA copy numbers were calculated relative to genomic DNA as previously described (Kaufmann et al., 2019; Miller et al., 2003).

### Mitochondria fractionation and transfer

Mitochondria/cytosol fractionation was performed using the mitochondria/cytosol fractionation kit (Abcam) according to the manufacturer's instructions. Mitochondria were transferred as described previously (Kim et al., 2018).

### Cell death assay

Cells were seeded into cell culture dishes and rested overnight. The medium was replaced by DBPS (+CaCl₂ +MgCl₂) containing 5 µg/ml Hoechst 33342 and 1 µg/ml propidium iodide (PI). Repetitive images were acquired for all fields of view over 19 h for BMDMs and 48 h for TECs using Zeiss Axiovert 200M Inverted Fluorescence microscope with a cage incubator from Oko lab. First, cells were kept under normoxic conditions (21% O₂) for 2 h. Thereafter, the O₂ content was reduced to a hypoxic atmosphere containing 1% O₂. For the analysis of cell death, both Hoechst-positive and PI-positive nuclei were counted per image. The number of apoptotic cells over time was normalized to normoxic conditions.

### Microarray data

Gene expression dataset GSE30718 was downloaded from GEO (http://www.ncbi.nlm.nih.gov/geo) (Clough and Barrett, 2016) and contained microarray data sets from 28 AKI biopsies and 11 protocol biopsies (Famulski et al., 2012).

### Statistical Analysis

All results were confirmed in triplicates by at least two independent experiments. Results were calculated and presented as mean values. Statistical analyses were performed using GraphPad^{™} prism 8 and One-way ANOVA followed by Tukey post-test was used for three or more group comparisons with *p<0.05, **p<0.01, and ***p<0.001 considered statistically significant.

### RESULTS

### Phenotyping and long-term survival of mice with genetic Ybx3 deletion.

The genetically modified *Ybx3*^{*-*/*-*} animals have been phenotyped before (Lu et al., 2006). No defect in embryonic development or animal survival rates until age 12 months were observed. However, male mice were less fertile with reduced testis weight due to seminiferous tubule degradation and enhanced spermatocyte apoptosis (Lu et al., 2006). Our main interest was the phenotyping of kidneys in *Ybx3*^{*-*/*-*} animals up to 2 years of age. Mortality rates were similar over 2 years for *Ybx3*^{+/+} and *Ybx3*^{-/-} mice (data not shown). Compared to wild type animals, a significantly lower body weight became apparent in *Ybx3*^{*-*/*-*} mice, which however remained within the normal range of 20-40 g **(****Figure 1A****).** Reduced food and water intake **(****Figure 1B****)** indicated a relative fasting state. The nutrient and energy sensor AMP-activated protein kinase (AMPK) was abundantly detected as phosphorylated protein. Increased phosphorylation of AMPKα was observed in kidney lysates from mice with heterozygous or homozygous *Ybx3* knockout **(****Figures 1C-D**). Reduced body weights did not affect rectal core body temperature, an indicator of energy consumption (data not shown). Furthermore, kidney function assessments (plasma creatinine, GFR, BUN), urine analyses (osmolality, pH, albuminuria and proteinuria), complete blood count and blood cell composition were similar in wild type, *Ybx3*^{+/-} and *Ybx3*^{-/-} animals (data not shown). Immunohistochemistry in wild type animals revealed DbpA positivity in smooth muscle cells, podocytes and tubular cells **(****Figure 1E****),** which corroborated with the Healthy Mouse Datasets (Wu et al., 2019) **(****Figure 1F****).** Western blot analyses of kidney lysates demonstrated less DbpA_a and DbpA_b expression in *Ybx3*^{+/-} animals, almost no DbpA isoforms were detected in *Ybx3*^{-/-} animals (**Figures 1G-H**). DbpA_a and DbpA_b were detected in healthy liver tissue of mice (data not shown). Given the report by Lima et al. describing high level *Ybx3* expression in cycling tubular cells during kidney ontogeny (Lima et al., 2010), we suspected a severe tubular cell defect in *Ybx3* deficient animals. Contrary to these expectations, PAS staining of *Ybx3*^{*-*/*-*} animals confirmed normal glomerular and tubular structures in the cortex (C), and similar tubular structures in the outer (OM) and inner medulla (IM) (data not shown). Immunohistochemistry for key proteins of water reabsorption (PC, principal cells) and pH regulation (IC, intercalated cells) revealed no differences in the average PC and IC numbers along the nephrons in the cortex, OM and IM of *Ybx3*^{*+*/*-*} and *Ybx3*^{*-*/*-*} mice (**Figures 1I-J**). These data were confirmed by Western blot analyses of PC (Aqp2, glycosylated Aqp2) and IC marker proteins (Atp6v1b1, CA2, AE1), as well as proximal tubular proteins (Aqp1, glycosylated Aqp1) of kidney cortex and medulla tissue lysates (data not shown). Merely transcript numbers of *Aqp2* were decreased in the kidney cortex of *Ybx3*^{-/-} mice compared to *Ybx3*^{+/+} and *Ybx3*^{+/-} animals (**Figures 1K-L**).

Taken together, our data demonstrated an unrestricted life expectancy with normal kidney function in *Ybx3*^{*-*/*-*} mice. Thus, DbpA is dispensable for kidney development and tissue homeostasis under healthy conditions. However, subtle differences exist in *Aqp2* expression. The lower body weight of the animals and the detection of abundant pAMPKα suggested unknown functions related to energy homeostasis.

### Tubular expression of DbpA inversely correlates with the mitochondrial oxygen consumption rate and glycolysis.

Kidneys are in demand of a large amount of energy for cell respiration in form of ATP, which is provided through mitochondrial respiration. In request to identify differential regulated pathways in *Ybx3*^{-/-} animals, kidneys from healthy mice were harvested to generate Agilent MicroArray libraries **(****Figure 2A****).** 364 differentially expressed genes were identified **(****Figure 2B****).** Gene set enrichment analysis revealed that the most significantly upregulated genes in the kidneys of *Ybx3*^{-/-} mice related to metabolic processes (**Figures 2C-D**). Functional metabolic analyses of TECs revealed increased mitochondrial respiration in terms of oxygen consumption rates (OCR) (**Figures 2E-F**) and glycolysis (**Figures 2G-I**). This indicates higher metabolic activity and increased energy supply in *Ybx3* deficient animals. In bone marrow-derived macrophages (BMDMs) these changes were not detected (data not shown), suggesting a tubular cell-specific effect of DbpA on metabolic functions. Furthermore, transfer of mitochondria isolated from *Ybx3*^{*-*/*-*} TECs into *Ybx3*^{+/+} TECs increased the metabolic activity **(****Figure 2J****).** Successful transfers of mitochondria into the recipient cells was confirmed by live cell imaging and overlay of fluorescent signals **(****Figure 2K****).** Moreover, increased metabolic activity of wild type BMDMs after the transfer of mitochondria isolated from *Ybx3*^{-/-} BMDMs were obtained (data not shown). In summary, these data revealed that genetic deletion of *Ybx3* in TECs increased the basal/maximal respiration and glycolytic activity by upregulating genes related to metabolic processes. Transfer of isolated mitochondria from *Ybx3*^{-/-} TECs into *Ybx3*^{+/+} TECs increased the OCR.

### DbpA co-localizes with mitochondrial marker proteins.

Based on the differences in the metabolic activities of TECs, we next analyzed mitochondrial mass by MitoTracker fluorescence intensities and mitochondrial DNA (mtDNA) copy numbers of isolated TECs **(****Figures 3A-B**). The total mitochondrial mass and relative transcripts levels of genes involved in mitochondrial gene expression, genome replication and biogenesis, such as *Tfam, Ppargclb* and *Pprcl* were unaffected by genetic *Ybx3* deletion **(****Figure 3C****).** Furthermore, electron microscopic analysis of healthy kidney tissues of mice with different genetic backgrounds revealed intact outer and inner mitochondrial membranes **(****Figure 3D****),** while the mitochondrial membrane potential was significantly increased in TECs isolated from *Ybx3*^{*-*/*-*} mice compared to wild type animals **(****Figure 3E****).** Based on the experimental protocol with isolation of TECs from kidneys of mice and disruption of structural components, it can be assumed that the primary cells were exposed to ischemic stress during the isolation process. In contrast, BMDMs from *Ybx3*^{+/+}*, Ybx3*^{+/-} and *Ybx3*^{*-*/*-*} mice showed no significant differences in MitoTracker fluorescence intensity and mtDNA copy number and mitochondrial membrane potential (data not shown). Western blot analysis of proteins related to the electron transport chain (ETC) revealed increased expression of ATP5A (ETC complex V) and UQCRC2 (ETC complex III) in TECs of *Ybx3*^{*-*/*-*} mice, MTCO1 (ETC complex IV) and SDHB (ETC complex II) were less expressed in *Ybx3*^{-/-} cells **(****Figures 3F-G**). In addition to the already described cytoplasmic, nuclear and cell-membrane localization (interaction partner of the tight junction protein Zonula occludens-1, (Balda and Matter, 2000)), we identified DbpA co-localization with mitochondrial marker proteins in TECs derived from *Ybx3*^{+/+} and *Ybx3*^{+/-} mice. Western blot analyses detected DbpA in the cytoplasm/nuclear fraction as well as in the mitochondrial fractions **(****Figure 3H****).** These data were verified by immunofluorescence staining of TECs, where a co-localization of DbpA with mitochondria fluorescence dye (MitoTracker) was clearly visible in cells derived from *Ybx3*^{+/+} and *Ybx3*^{+/-} mice, but not in *Ybx3*^{*-*/*-*} cells **(****Figure 3I****).** Taken together, our results clearly demonstrated that DbpA is a mitochondrial protein in TECs and in its absence the mitochondrial membrane potential increases.

### Following IRI upregulated DbpA expression confers tubular cell damage.

Epithelial cells form a boundary in the tubular barrier system with immediate contact to urine. Because of their prominent role in the maintenance of fluid and acid-base homeostasis, TECs have a high energy requirement and are especially sensitive to ischemia. *Ybx3* transcripts were present in kidney tissue from protocol transplant biopsies in humans and significant upregulation was observed in those with AKI (Famulski et al., 2012). Notably, another prominent cold shock protein YB-1 (*Ybx1*) was also upregulated as reported recently (Wang et al., 2023) **(****Figure 4A****).**

At the protein level, a 3-fold upregulation of DbpA_a and DbpA_b was detected in kidney lysates and kidney sections 1 day following IRI **(****Figures 4B****),** which was transient and no longer detected on day 28 post IRI **(****Figures 4C-F**). Survival analysis over 28 d following IRI revealed 100% survival of *Ybx3*^{*-*/*-*} mice, 90% of *Ybx3*^{*+*/}*⁻,* whereas 70% of *Ybx3*^{+/+} mice survived **(****Figure 5A****).** The overall appearance of kidneys from *Ybx3*^{+/+} and *Ybx3*^{+/-} animals 1 day following IRI was in deep red color, whereas kidneys from *Ybx3*^{-/-} animals had a pale brownish color, similar to healthy controls **(****Figure 5B****).** Wild type animals showed increased tubular injury 1 d following IRI, which was diminished or absent in *Ybx3*^{+/-} and *Ybx3*^{-/-} mice **(****Figures 5C-D**). Correspondingly, kidney function (GFR) remained normal in *Ybx3*^{-/-} animals (Figure 5E). Tubular cell damage marker neutrophil gelatinase-associated lipocalin (NGAL) was strongly upregulated short-term following IRI in *Ybx3*^{+/+}*,* but minimally in knockout animals and primary tubular cell cultures **(****Figures 5F-I**). The upregulated signaling molecules pERK1/2 (T²⁰²/Y²⁰⁴) and pJNK (T¹⁸³/Y¹⁸⁵) were still detected 28 d following IRI in *Ybx3*^{+/+} kidney lysates, whereas they were less expressed in *Ybx3*^{+/-} and *Ybx3*^{-/-} mice. The expression levels of pp38 (T¹⁸⁰/Y¹⁸²) and pAKT (S⁴⁷³) were elevated 1 day following IRI and returned to basal levels after 28 days (data not shown). Overall, DbpA mediates tubular cell damage in AKI and the absence of the protein is linked with cell protection.

### Ybx3^{-/-} animals do not develop an inflammatory and fibrotic response.

In *Ybx3*^{+/+} animals transient immune cell infiltration into the kidney was induced by IRI. However, in *Ybx3*^{-/-} mice infiltrating T cell, neutrophil, macrophage and monocyte cell numbers remained low following IRI **(****Figures 6A****).** Circulating leukocytes were similar in the genotypes following IRI (data not shown). ER stress was detected in *Ybx3*^{+/+} and *Ybx3*^{+/-} but not in *Ybx3*^{*-* /*-*} animals, as indicated by increased ER stress transcript levels **(****Figures 6B****)** as well as ATF6 and ATF4 protein expression levels (**Figures 6C-D**). Long-term analysis 28 d following IRI revealed an absent fibrotic response in *Ybx3*^{*-*/*-*} animals. The fibrosis marker proteins αSMA, Tenascin-C **(****Figures 6E-F**), matrisome related transcripts **(****Figures 6G****)** and collagens **(****Figures 6H-I**) were not upregulated in *Ybx3*^{-/-} animals. In summary, the absence of DbpA protects from inflammation and fibrosis.

### Increased antioxidant activity provides the basis for the protective mechanism.

The levels of mitochondrial reactive oxygen species (mROS) levels in TECs of *Ybx3*^{+/+}*, Ybx3*^{+/-} and *Ybx3*^{-/-} mice were similar **(****Figure 7A****),** whereas the antioxidant enzymes GPX4 and SOD2 were upregulated at baseline in TECs obtained from *Ybx3*^{-/-} **(****Figures 7B-C**). PTEN-induced kinase 1 (PINK1) protects cells from mitochondrial dysfunction by inducing mitophagy. We detected an upregulation of PINK1 in *Ybx3*^{*-*/*-*} TECs under normoxic and hypoxic (OGD) conditions **(****Figures 7B-D**). Unexpectedly, no apoptosis-related transcript regulation was detected **(****Figure 7E****).** These findings were corroborated by an *in vitro* assay of TEC apoptosis and nuclear staining with Hoechst and PI. Here, the number of apoptotic cells was similar in the genotypes after hypoxia **(****Figure 7F****).** In BMDMs, these effects were similar (data not shown). However, transcript analysis showed upregulation of ferroptosis-related genes 1 d following IRI in kidneys of wild type animals, which was less evident in the kidneys of *Ybx3*^{-/-} animals **(****Figure 7G****).** The most differentially expressed transcript *HMOX1* was also enhanced expressed on the protein level in wild type kidney lysates 1 d following IRI (**Figures 7H-I**). Furthermore, kidney sections of wild type and *Ybx3*^{+/-} animals revealed more positive staining of the lipid peroxidation marker 4-hydroxynonenal (4HNE) especially at the corticomedullary junction **(****Figure 7J****),** suggesting increased ferroptosis. In conclusion, *Ybx3*^{-/-} TECs were metabolically more active at baseline and better adapted to hypoxic conditions due to their upregulated antioxidant functions, which probably neutralizes mROS. Since mROS is the main cause of ferroptosis induction, *Ybx3*^{-/-} animals were protected from transient hypoxic conditions.

### REFERENCES

Balda, M.S., and Matter, K. (2000). The tight junction protein ZO-1 and an interacting transcription factor regulate ErbB-2 expression. The EMBO journal 19, 2024-2033.
Balda, M.S., and Matter, K. (2009). Tight junctions and the regulation of gene expression. Biochimica et biophysica acta 1788, 761-767.
Bargou, R.C., Jürchott, K., Wagener, C., Bergmann, S., Metzner, S., Bommert, K., Mapara, M.Y., Winzer, K.J., Dietel, M., Dörken, B., et al. (1997). Nuclear localization and increased levels of transcription factor YB-1 in primary human breast cancers are associated with intrinsic MDR1 gene expression. Nature medicine 3, 447-450.
Chatterjee, M., Rancso, C., Stühmer, T., Eckstein, N., Andrulis, M., Gerecke, C., Lorentz, H., Royer, H.D., and Bargou, R.C. (2008). The Y-box binding protein YB-1 is associated with progressive disease and mediates survival and drug resistance in multiple myeloma. Blood 111, 3714-3722.
Clough, E., and Barrett, T. (2016). The Gene Expression Omnibus Database. Methods in molecular biology (Clifton, NJ) 1418, 93-110.
Dahl, E., En-Nia, A., Wiesmann, F., Krings, R., Djudjaj, S., Breuer, E., Fuchs, T., Wild, P.J., Hartmann, A., Dunn, S.E., et al. (2009). Nuclear detection of Y-box protein-1 (YB-1) closely associates with progesterone receptor negativity and is a strong adverse survival factor in human breast cancer. BMC cancer 9, 410.
De Chiara, L., Conte, C., Semeraro, R., Diaz-Bulnes, P., Angelotti, M.L., Mazzinghi, B., Molli, A., Antonelli, G., Landini, S., Melica, M.E., et al. (2022). Tubular cell polyploidy protects from lethal acute kidney injury but promotes consequent chronic kidney disease. Nat Commun 13, 5805.
Dong, W., Wang, H., Shahzad, K., Bock, F., Al-Dabet, M.M., Ranjan, S., Wolter, J., Kohli, S., Hoffmann, J., Dhople, V.M., et al. (2015). Activated Protein C Ameliorates Renal Ischemia-Reperfusion Injury by Restricting Y-Box Binding Protein-1 Ubiquitination. J Am Soc Nephrol 26, 2789-2799.
Doulamis, I.P., Guariento, A., Duignan, T., Kido, T., Orfany, A., Saeed, M.Y., Weixler, V.H., Blitzer, D., Shin, B., Snay, E.R., et al. (2020). Mitochondrial transplantation by intra-arterial injection for acute kidney injury. American journal of physiology Renal physiology 319, F403-f413.
Duann, P., and Lin, P.H. (2017). Mitochondria Damage and Kidney Disease. Advances in experimental medicine and biology 982, 529-551.
Famulski, K.S., de Freitas, D.G., Kreepala, C., Chang, J., Sellares, J., Sis, B., Einecke, G., Mengel, M., Reeve, J., and Halloran, P.F. (2012). Molecular phenotypes of acute kidney injury in kidney transplants. J Am Soc Nephrol 23, 948-958.
Gao, P., Yan, Z., and Zhu, Z. (2020). Mitochondria-Associated Endoplasmic Reticulum Membranes in Cardiovascular Diseases. Frontiers in cell and developmental biology 8, 604240.
Gibbert, L., Hermert, D., Wang, J., D, M.B., Alidousty, C., Neusser, M., Cohen, C.D., Gröne, E., Macheleidt, I., Rauen, T., et al. (2018). YB-1 increases glomerular, but decreases interstitial fibrosis in CNI-induced nephropathy. Clinical immunology (Orlando, Fla) 194, 67-74. Giménez-Bonafé, P., Fedoruk, M.N., Whitmore, T.G., Akbari, M., Ralph, J.L., Ettinger, S., Gleave, M.E., and Nelson, C.C. (2004). YB-1 is upregulated during prostate cancer tumor progression and increases P-glycoprotein activity. The Prostate 59, 337-349.
Guariento, A., Piekarski, B.L., Doulamis, I.P., Blitzer, D., Ferraro, A.M., Harrild, D.M., Zurakowski, D., Del Nido, P.J., McCully, J.D., and Emani, S.M. (2021). Autologous mitochondrial transplantation for cardiogenic shock in pediatric patients following ischemia-reperfusion injury. The Journal of thoracic and cardiovascular surgery 162, 992-1001.
Hall, A.M., Unwin, R.J., Parker, N., and Duchen, M.R. (2009). Multiphoton imaging reveals differences in mitochondrial function between nephron segments. J Am Soc Nephrol 20, 1293-1302.
Hanssen, L., Alidousty, C., Djudjaj, S., Frye, B.C., Rauen, T., Boor, P., Mertens, P.R., van Roeyen, C.R., Tacke, F., Heymann, F., et al. (2013). YB-1 is an early and central mediator of bacterial and sterile inflammation in vivo. Journal of immunology (Baltimore, Md : 1950) 191, 2604-2613.
Hohlfeld, R., Brandt, S., Bernhardt, A., Gorny, X., Schindele, D., Jandrig, B., Schostak, M., Isermann, B., Lindquist, J.A., and Mertens, P.R. (2018). Crosstalk between Akt signaling and cold shock proteins in mediating invasive cell phenotypes. Oncotarget 9, 19039-19049.
Jayavelu, A.K., Schnöder, T.M., Perner, F., Herzog, C., and Meiler, A. (2020). Splicing factor YBX1 mediates persistence of JAK2-mutated neoplasms. 588, 157-163.
Kato, M., Wang, L., Putta, S., Wang, M., Yuan, H., Sun, G., Lanting, L., Todorov, I., Rossi, J.J., and Natarajan, R. (2010). Post-transcriptional up-regulation of Tsc-22 by Ybx1, a target of miR-216a, mediates TGF-{beta}-induced collagen expression in kidney cells. The Journal of biological chemistry 285, 34004-34015.
Kaufmann, U., Kahlfuss, S., Yang, J., Ivanova, E., Koralov, S.B., and Feske, S. (2019). Calcium Signaling Controls Pathogenic Th17 Cell-Mediated Inflammation by Regulating Mitochondrial Function. Cell Metab 29, 1104-1118.e1106.
Khwaja, A. (2012). KDIGO clinical practice guidelines for acute kidney injury. Nephron Clin Pract 120, c179-184.
Kim, M.J., Hwang, J.W., Yun, C.K., Lee, Y., and Choi, Y.S. (2018). Delivery of exogenous mitochondria via centrifugation enhances cellular metabolic function. Scientific reports 8, 3330.
Klomjit, N., and Ungprasert, P. (2022). Acute kidney injury associated with non-steroidal anti-inflammatory drugs. European journal of internal medicine 101, 21-28.
Konari, N., Nagaishi, K., Kikuchi, S., and Fujimiya, M. (2019). Mitochondria transfer from mesenchymal stem cells structurally and functionally repairs renal proximal tubular epithelial cells in diabetic nephropathy in vivo. Scientific reports 9, 5184.
Lima, W.R., Parreira, K.S., Devuyst, O., Caplanusi, A., N'Kuli, F., Marien, B., Van Der Smissen, P., Alves, P.M., Verroust, P., Christensen, E.I., et al. (2010). ZONAB promotes proliferation and represses differentiation of proximal tubule epithelial cells. J Am Soc Nephrol 21, 478-488.
Lindquist, J.A., and Mertens, P.R. (2018). Cold shock proteins: from cellular mechanisms to pathophysiology and disease. Cell communication and signaling : CCS 16, 63.
Lu, Z.H., Books, J.T., and Ley, T.J. (2006). Cold shock domain family members YB-1 and MSY4 share essential functions during murine embryogenesis. Mol Cell Biol 26, 8410-8417.
Malek, M., and Nematbakhsh, M. (2015). Renal ischemia/reperfusion injury; from pathophysiology to treatment. Journal of renal injury prevention 4, 20-27.
Martinez-Klimova, E., Aparicio-Trejo, O.E., Gómez-Sierra, T., Jiménez-Uribe, A.P., Bellido, B., and Pedraza-Chaverri, J. (2020). Mitochondrial dysfunction and endoplasmic reticulum stress in the promotion of fibrosis in obstructive nephropathy induced by unilateral ureteral obstruction. 46, 716-733.
Matter, K., and Balda, M.S. (2007). Epithelial tight junctions, gene expression and nucleo-junctional interplay. Journal of cell science 120, 1505-1511.
Mehta, R.L., Cerdá, J., Burdmann, E.A., Tonelli, M., Garcia-Garcia, G., Jha, V., Susantitaphong, P., Rocco, M., Vanholder, R., Sever, M.S., et al. (2015). International Society of Nephrology's 0by25 initiative for acute kidney injury (zero preventable deaths by 2025): a human rights case for nephrology. Lancet (London, England) 385, 2616-2643.
Miller, F.J., Rosenfeldt, F.L., Zhang, C., Linnane, A.W., and Nagley, P. (2003). Precise determination of mitochondrial DNA copy number in human skeletal and cardiac muscle by a PCR-based assay: lack of change of copy number with age. Nucleic acids research 31, e61.
Mukherjee, M., deRiso, J., Otterpohl, K., Ratnayake, I., Kota, D., Ahrenkiel, P., Chandrasekar, I., and Surendran, K. (2019). Endogenous Notch Signaling in Adult Kidneys Maintains Segment-Specific Epithelial Cell Types of the Distal Tubules and Collecting Ducts to Ensure Water Homeostasis. J Am Soc Nephrol 30, 110-126.
Naba, A., Clauser, K.R., Ding, H., Whittaker, C.A., Carr, S.A., and Hynes, R.O. (2016). The extracellular matrix: Tools and insights for the "omics" era. Matrix biology : journal of the International Society for Matrix Biology 49, 10-24.
National Clinical Guideline, C. (2013). National Institute for Health and Clinical Excellence: Guidance. In Acute Kidney Injury: Prevention, Detection and Management Up to the Point of Renal Replacement Therapy (London: Royal College of Physicians (UK) Copyright © 2013, National Clinical Guideline Centre.).
Ni, L., and Yuan, C. (2021). The Mitochondrial-Associated Endoplasmic Reticulum Membrane and Its Role in Diabetic Nephropathy. Oxid Med Cell Longev 2021, 8054817.
Ostermann, M., Bellomo, R., Burdmann, E.A., Doi, K., Endre, Z.H., Goldstein, S.L., Kane-Gill, S.L., Liu, K.D., Prowle, J.R., Shaw, A.D., et al. (2020). Controversies in acute kidney injury: conclusions from a Kidney Disease: Improving Global Outcomes (KDIGO) Conference. Kidney Int 98, 294-309.
Pan, W., Wang, L., Zhang, X.F., Zhang, H., and Zhang, J. (2019). Hypoxia-induced microRNA-191 contributes to hepatic ischemia/reperfusion injury through the ZONAB/Cyclin D1 axis. 26, 291-305.
Peired, A.J., and Melica, M.E. (2021). Molecular Mechanisms of Renal Progenitor Regulation: How Many Pieces in the Puzzle? 10.
Randles, M.J., Lausecker, F., Kong, Q., Suleiman, H., and Reid, G. (2021). Identification of an Altered Matrix Signature in Kidney Aging and Disease. 32, 1713-1732.
Rovin, B.H., Adler, S.G., Barratt, J., Bridoux, F., Burdge, K.A., Chan, T.M., Cook, H.T., Fervenza, F.C., Gibson, K.L., Glassock, R.J., et al. (2021). Executive summary of the KDIGO 2021 Guideline for the Management of Glomerular Diseases. Kidney Int 100, 753-779.
Ruan, Y.C., Wang, Y., Da Silva, N., Kim, B., Diao, R.Y., Hill, E., Brown, D., Chan, H.C., and Breton, S. (2014). CFTR interacts with ZO-1 to regulate tight junction assembly and epithelial differentiation through the ZONAB pathway. Journal of cell science 127, 4396-4408.
Schock-Kusch, D., Sadick, M., Henninger, N., Kraenzlin, B., Claus, G., Kloetzer, H.M., Weiss, C., Pill, J., and Gretz, N. (2009). Transcutaneous measurement of glomerular filtration rate using FITC-sinistrin in rats. Nephrology, dialysis, transplantation : official publication of the European Dialysis and Transplant Association - European Renal Association 24, 2997-3001.
Scott, N.E., Rogers, L.D., Prudova, A., Brown, N.F., Fortelny, N., Overall, C.M., and Foster, L.J. (2017). Interactome disassembly during apoptosis occurs independent of caspase cleavage. 13, 906.
Seufert, L., and Benzing, T. (2022). RNA-binding proteins and their role in kidney disease. 18, 153-170.
Tonnus, W., and Meyer, C. (2021). Dysfunction of the key ferroptosis-surveilling systems hypersensitizes mice to tubular necrosis during acute kidney injury. 12, 4402.
van Roeyen, C.R., Eitner, F., Martinkus, S., Thieltges, S.R., Ostendorf, T., Bokemeyer, D., Liischer, B., Liischer-Firzlaff, J.M., Floege, J., and Mertens, P.R. (2005). Y-box protein 1 mediates PDGF-B effects in mesangioproliferative glomerular disease. J Am Soc Nephrol 16, 2985-2996.
Wang, J., Liu, X., Gu, Y., Gao, Y., Jankowski, V., Was, N., Leitz, A., Reiss, L.K., Shi, Y., Cai, J., et al. (2023). DNA binding protein YB-1 is a part of the neutrophil extracellular trap mediation of kidney damage and cross-organ effects. Kidney international.
Wolffe, A.P. (1994). Structural and functional properties of the evolutionarily ancient Y-box family of nucleic acid binding proteins. BioEssays : news and reviews in molecular, cellular and developmental biology 16, 245-251.
Wolffe, A.P., Tafuri, S., Ranjan, M., and Familari, M. (1992). The Y-box factors: a family of nucleic acid binding proteins conserved from Escherichia coli to man. New Biol 4, 290-298.
Wu, H., Kirita, Y., Donnelly, E.L., and Humphreys, B.D. (2019). Advantages of Single-Nucleus over Single-Cell RNA Sequencing of Adult Kidney: Rare Cell Types and Novel Cell States Revealed in Fibrosis. J Am Soc Nephrol 30, 23-32.
Zhu, C., Sauter, E., Schreiter, A., van Roeyen, C.R., Ostendorf, T., Floege, J., Gembardt, F., Hugo, C.P., Isermann, B., Lindquist, J.A., et al. (2016). Cold Shock Proteins Mediate GN with Mesangioproliferation. J Am Soc Nephrol 27, 3678-3689.

## Claims

1. A DNA-binding protein-A (DbpA) modulating compound for use in the treatment of an acute kidney injury (AKI).

2. The DbpA modulating compound for use of claim 1, wherein the DbpA modulating compound is a DbpA binding/inhibiting molecule.

3. The DbpA modulating compound for use of claim 2, wherein the DbpA binding/inhibiting molecule is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities.

4. The DbpA modulating compound for use of claim 3, wherein the miRNA is miRNA-191 or the antibody is an anti-DbpA antibody.

5. The DbpA modulating compound for use of any one of claims 1 to 4, wherein the AKI is selected from the group consisting of prerenal AKI, intrarenal AKI, and postrenal AKI.

6. A combination of a DbpA modulating compound and a drug different from a DbpA modulating compound for use in the treatment of an acute kidney injury (AKI).

7. The combination for use of claim 6, wherein
(i) the DbpA modulating compound is a DbpA binding/inhibiting molecule, and/or
(ii) the drug different from a DbpA modulating compound is selected from the group consisting of oxygen-free radical scavengers, angiotensin II and adenosine receptor antagonists, Alkaline phosphatase, sphingosine 1 phosphate analogues, dipeptidylpeptidase-4 inhibitors.

8. The combination for use of claim 7, wherein the DbpA binding/inhibiting molecule is selected from the group consisting of a miRNA (miRNA), a small interfering RNA (siRNA), a short hairpin RNA (shRNA), an antisense 2'-O-methyl (2'-OMe) oligoribonucleotide, an antibody or a fragment thereof, and a (synthetic) compound interfering with DbpA activities.

9. The combination for use of claim 8, wherein
the miRNA is miRNA-191, or
the antibody is an anti-DbpA antibody.

10. The combination for use of any one of claims 7 to 9, wherein the oxygen-free radical scavengers are selected from the group consisting of α-lipoic acid, curcumin, sodium-2-mercaptoethane sulphonate, propofol, and selenium.

11. A pharmaceutical composition comprising a DNA-binding protein-A (DbpA) of any one of claims 1 to 5 or a combination of any one of claims 6 to 10 for use in the treatment of an acute kidney injury (AKI).

12. A method for treating an acute kidney injury (AKI) in an individual comprising the step of: administering (an effective amount of) a DNA-binding protein-A (DbpA) modulating compound to an individual in need thereof.

13. A method for treating an acute kidney injury (AKI) in an individual comprising the step of: administering (an effective amount of) a combination of a DbpA modulating compound and a drug different from a DbpA modulating compound to an individual in need thereof.

14. Use of a DNA-binding protein-A (DbpA) modulating compound for the manufacture of a medicament/drug for the treatment of an acute kidney injury (AKI).

15. Use of a combination of a DbpA modulating compound and a drug different from a DbpA modulating compound for the manufacture of a medicament/drug for the treatment of an acute kidney injury (AKI).
